(19) Europäisches Patentamt / European Patent Office / Office européen des brevets

(11) **EP 3 437 671 B1**

(12) **EUROPEAN PATENT SPECIFICATION**

(45) Date of publication and mention
of the grant of the patent:
**16.02.2022 Bulletin 2022/07**

(21) Application number: **17775083.3**

(22) Date of filing: **28.03.2017**

(51) International Patent Classification (IPC):
**B01J 20/28** $^{(2006.01)}$     **B01J 20/26** $^{(2006.01)}$
**A61M 1/36** $^{(2006.01)}$     **A61M 1/34** $^{(2006.01)}$

(52) Cooperative Patent Classification (CPC):
**B01J 20/28023; A61M 1/34; A61M 1/36;**
**B01J 20/261; B01J 20/262; B01J 20/28004;**
**B01J 20/28057; B01J 20/28078;** B01J 2220/58

(86) International application number:
**PCT/JP2017/012631**

(87) International publication number:
**WO 2017/170546 (05.10.2017 Gazette 2017/40)**

(54) **FIBER MATERIAL AND PURIFICATION COLUMN**

FASERMATERIAL UND REINIGUNGSKOLONNE

MATÉRIAU FIBREUX ET COLONNE DE PURIFICATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **30.03.2016 JP 2016068094**
**31.03.2016 JP 2016071297**

(43) Date of publication of application:
**06.02.2019 Bulletin 2019/06**

(73) Proprietor: **Toray Industries, Inc.**
**Tokyo 103-8666 (JP)**

(72) Inventors:
• **FUJIEDA, Hiroaki**
**Otsu-shi**
**Shiga 520-8558 (JP)**
• **UENO, Yoshiyuki**
**Otsu-shi**
**Shiga 520-8558 (JP)**
• **YAMADA, Masayuki**
**Otsu-shi**
**Shiga 520-8558 (JP)**

(74) Representative: **Mewburn Ellis LLP**
**Aurora Building**
**Counterslip**
**Bristol BS1 6BX (GB)**

(56) References cited:
**JP-A- H0 941 221**     **JP-A- S6 215 321**
**JP-A- S61 207 638**     **US-A1- 2009 275 874**
**US-A1- 2013 071 659**     **US-A1- 2015 374 898**

**Description**

TECHNICAL FIELD

The present invention relates to a fibrous material that enables efficient adsorption of substances to be removed in a fluid that is to be treated, and to a purification column incorporating the fibrous material.

BACKGROUND ART

Traditionally, porous beads have often been used as a form of adsorption material for use in a purification column that adsorbs and removes substances to be removed in a fluid that is to be treated. The reason is that an adsorbent in the form of beads can uniformly fill the space within an adsorption column, which advantageously enables to easily design a column in which a less uneven distribution of blood flow is achieved. Moreover, as a means of improving the adsorption performance, the volume-specific surface area of an adsorbent is increased. However, when an adsorbent is in the form of beads and the volume-specific surface area of the adsorbent is increased by reducing the bead diameter, the space between beads is reduced. Then, the flow resistance and the pressure loss are increased, which causes difficulty in allowing a fluid that is to be treated to flow. Moreover, beads that are used as an adsorbent are usually spherical and therefore essentially have a smaller volume-specific surface area relative to those of other geometric shapes. In other words, even if beads still leave adsorption space inside, those adsorption sites sometimes cannot be effectively used.

A form of adsorption material other than beads can be a form of fiber, and it is contemplated that commonly used fibers having a round cross section are used as the adsorption material. Examples of the fiber-form adsorption material include multiple fibers in a straight form or a knitted form which have been installed into a column in parallel with the axial direction extending between openings on both sides of the column casing.

PRIOR ART DOCUMENTS

PATENT DOCUMENTS

The inventions relating to purification columns in which hollow fibers or solid fibers are incorporated have been disclosed to date (Patent Document 1 and 2).

In the field of clothing, methods of blending various types of fibers having different cross-sectional shapes have been disclosed to date, as described in Patent Document 3 to 5, for the purpose of enhancing the puffy, or bulky, texture. In the field of, for example, dialyzer technology, a technique as described in Patent Document 6 and 7 has been invented, in which fibers are crimped to prevent overlap and adhesion between neighboring fibers.

Moreover, Patent Document 8 describes an invention to prevent uneven distribution of fluid flow by a combination of hollow fiber membranes having a round cross-sectional shape and spacer filaments having a different cross-sectional shape. Patent Document 9 describes an absorbent which can remove cells present in blood including activated leukocytes such as granulocytes and monocytes and cancer cells; the absorbent can also remove cytokines which facilitates the activation of the remaining cells. Patent Document 10 describes a blood purification column that includes an adsorbent and a casing whose two ends are open ends, wherein the adsorbent is housed inside the casing. One of the two casing ends is a blood inflow-side portion and the other is a blood outflow-side end portion. A filter is disposed at the blood inflow-side end portion and/or the blood outflow-side end portion of the casing.

Patent Document 1: JP 2011-156022 A
Patent Document 2: JP 2010-148851 A
Patent Document 3: JP 2002-220758 A
Patent Document 4: JP 2004-263341 A
Patent Document 5: JP 2002-194621 A
Patent Document 6: JP 2012-115743 A
Patent Document 7: JP 2008-155009 A
Patent Document 8: JP 2000-225304 A
Patent Document 9: US 2009/275874
Patent Document 10: US 2015/071659

Non Patent Document

Non Patent Document 1: Kazuhiko Ishikiriyama et al.; JOURNAL OF COLLOID AND INTERFACE SCIENCE, VOL. 171, 103-111 (1995)

## SUMMARY OF THE INVENTION

### PROBLEMS TO BE SOLVED BY THE INVENTION

However, the fibers used in Patent Document 1 and 2 had a round cross-sectional shape and the resulting adsorption material had a small volume-specific surface area, which in turn resulted in low adsorption performance. Then, a method in which fibers having a cross-sectional shape other than round, namely, having a modified cross-sectional shape is used is contemplated. However, when the filling ratio of filaments in a purification column is increased for the purpose of increasing the adsorption performance of the purification column, the occupancy rate against a circumscribed circle (the cross-sectional area ratio of a fiber with a modified cross-sectional shape to the circumscribed circle of the fiber; described below) is reduced in modified cross-section fibers as compared with fibers having a round cross section, which causes neighboring filaments to overlap and adhere to each other and then to easily reduce the exposed surface area of the filaments.

The inventions described in Patent Document 6 and 7 have problems such as necessity of an additional step of crimping and breakage of filaments during the crimping step, and further has a problem of the crimp wave that tends to decrease the height over long-term storage or due to deterioration over time, which have not been preferable in terms of long-term stability. Also, the Patent Documents 3 to 5 did not describe that the inventions aimed at adsorbing substances to be removed in a fluid that is to be treated. Moreover, in the inventions, porous fibers were not used but filaments were heated for the purpose of improving, for example, the puffy texture and/or the light-weight texture by making use of the difference in shrinkage ratio between the filaments; however, if porous filaments having micropores are used, the porous structure may be destroyed by heat treatment or higher-order processing.

Moreover, in the invention described in Patent Document 8, when a fluid that is to be treated flows only inside hollow fiber membranes, the fluid that is to be treated contacts only with the inner surfaces of hollow fibers, whereas when a fluid that is to be treated flows only outside hollow fiber membranes, the fluid that is to be treated contacts only with the outer surfaces of spacer filaments and those of hollow fibers, which causes low adsorption performance in either case. Moreover, a method in which a fluid that is to be treated is continuously introduced into both the inside and outside of hollow fibers may also be recognized, but the method results in complication of the flow path for a fluid that is to be treated and leads to an increase in pressure loss and is therefore not preferable.

In one method, both ends of a casing are not provided with partition walls but sealed with, for example, meshes and a liquid to be treated flows both inside and outside hollow fibers, but it is difficult to distribute an equal volume of fluid to the inside and outside of the hollow fibers, which easily causes an uneven flow.

Moreover, for example, when blood as a fluid that is to be treated is introduced into a column and then the blood remaining in the column is returned to the body by using saline (the operation is sometimes referred to as "blood reinfusion"), it is concerned that particularly hollow fibers with a small inner diameter or flattened hollow fibers cause a phenomenon called blood retention, which refers to a large volume of blood remaining inside the hollow fibers during blood reinfusion, to occur. Therefore, the above-described method is not preferable.

In view of the above-described conventional technologies, a problem to be solved by the invention is to provide a fibrous material having an excellent ability to adsorb substances and to remove the adsorbed substances, and a purification column incorporating the fibrous material.

### MEANS FOR SOLVING THE PROBLEMS

A fibrous material according to the present invention is as disclosed in the claims 1-11.

The fibrous material comprises polymeric fiber blends having plural types of solid fibers having a common cross-sectional shape, wherein the composition ratio of each of at least two out of the plural types of fibers to the total of the fiber blends is not less than 5.0%, and when, among those plural types of fibers having a composition ratio of not less than 5.0%, fibers having the highest and the lowest surface area increase rates, which are given by the formula (1) below, are respectively designated as the $fiber_{(max)}$ and the $fiber_{(min)}$, the surface area increase rate of the $fiber_{(min)}$ is reduced by 3.0% or more as compared to that of the $fiber_{(max)}$, and the composition ratios of the $fiber_{(max)}$ and the $fiber_{(min)}$ to the total of the fiber blends are not less than 30.0% and not less than 8.0%, respectively, and the $fiber_{(max)}$ has (a) a surface area increase rate of not less than 1.20 and is (b) a porous fiber with a micropore specific surface area of not less than 10 $m^2/g$.

Surface area increase rate = (the circumference of a fiber cross section) / (the circumference of a circle having the same cross-sectional area as the fiber cross section) (1)

A purification column according to the present invention is as disclosed in claim 12.

The purification column comprises a plastic casing and the above-described fibrous material, in which the fibrous material is arranged in the plastic casing straight in the axial direction extending between openings on both sides of the casing, and an inlet port and an outlet port for a fluid that is to be treated are provided at either end of the plastic casing.

In the fibrous material of the present invention, the micropore specific surface area of the $\text{fiber}_{(max)}$ is not less than 10 $m^2/g$.

In the fibrous material of the present invention, the $\text{fiber}_{(min)}$ is preferably a porous fiber with a micropore specific surface area of not less than 5 $m^2/g$.

In the fibrous material of the present invention, the micropore specific surface area of the $\text{fiber}_{(min)}$ is preferably not less than 10 $m^2/g$.

In the fibrous material of the present invention, the occupancy rate *Sfo* against the circumscribed circle of the $\text{fiber}_{(min)}$, which is given by the formula (2) below, is not more than 0.90.

$$\text{Occupancy rate } \textit{Sfo} \text{ against a circumscribed circle} = \textit{Sf} / \textit{So} \qquad (2)$$

wherein *Sf* represents the cross-sectional area of a fiber cross section and *So* represents the area of the circumscribed circle of the fiber cross section.

In the fibrous material of the present invention, the modification degree *Do/Di* of the $\text{fiber}_{(min)}$ is not less than 1.1 and not more than 6.00, where *Do* represents the diameter of the circumscribed circle of a fiber cross section and *Di* represents the diameter of the inscribed circle of the fiber cross section.

In the fibrous material of the present invention, the surface area increase rate of the $\text{fiber}_{(min)}$ is preferably not less than 1.10.

In the fibrous material of the present invention, the fiber cross-sectional shape of the $\text{fiber}_{(min)}$ is preferably round or oval.

In the fibrous material of the present invention, the equivalent circle diameter of the $\text{fiber}_{(max)}$ is preferably not less than 10 $\mu$m and not more than 1,000 $\mu$m.

In the fibrous material of the present invention, the *Sfo* ratio Z obtained by dividing the occupancy rate *Sfo* against the circumscribed circle of the $\text{fiber}_{(min)}$ by the occupancy rate *Sfo* against the circumscribed circle of the $\text{fiber}_{(max)}$ is preferably not less than 0.20.

In the fibrous material of the present invention, the fiber diameter ratio Y obtained by dividing the equivalent circle diameter of the $\text{fiber}_{(max)}$ by the equivalent circle diameter of the $\text{fiber}_{(min)}$ is not less than 0.2 and not more than 10.0.

The fibrous material of the present invention is preferably composed of straight fibers.

In the fibrous material of the present invention, the fiber blend is preferably composed of two types of fibers having a common cross-sectional shape.

In the fibrous material of the present invention, the ratio between the $\text{fiber}_{(max)}$ and the $\text{fiber}_{(min)}$ is preferably from 10:1 to 1:2.

In the fibrous material of the present invention, the $\text{fiber}_{(max)}$ and the $\text{fiber}_{(min)}$ are preferably produced from the same raw material.

In the fibrous material of the present invention, the raw material is preferably an ester group-containing polymer.

The fibrous material of the present invention is preferably for use in medical applications.

The fibrous material of the present invention is preferably for use in purification column applications.

The fibrous material of the present invention preferably adsorbs $\beta_2$-microglobulin with an adsorption capacity of not less than 0.005 mg/cm3.

In the purification column of the present invention, the equivalent diameter of flow cross-section is not less than 10 $\mu$m and not more than 250 $\mu$m The equivalent diameter of flow cross-section (herein after, $D_p$) is obtained based on the following formula (11).

$$D_p = 4 \times ((D_{case}/2)^2 - (D_{fiber-max}/2)^2 \times N_{max} - (D_{fiber-min}/2)^2 \times N_{min}) / (D_{case} + D_{fiber-max} \times SA_{max}$$

$$\times N_{max} + D_{fiber-min} \times SA_{min} \times N_{min}) \qquad (11),$$

wherein in the formula (11), $D_{case}$ represents the inner diameter of a column casing, and $D_{fiber-max}$ represents the equivalent circle diameter of a $\text{fiber}_{(max)}$, $D_{fiber-min}$ represents the equivalent circle diameter of a $\text{fiber}_{(min)}$, $N_{max}$ represents the number of $\text{fibers}_{(max)}$, $N_{min}$ represents the number of $\text{fibers}_{(min)}$, $SA_{max}$ represents the surface area increase rate of a $\text{fiber}_{(max)}$ and $SA_{min}$ represents the surface area increase rate of a $\text{fiber}_{(min)}$.

In the purification column of the present invention, the pressure loss achieved by allowing bovine blood to flow at a flow rate of 200 mL/min is from 0.5 to 30 kPa.

EFFECTS OF THE INVENTION

According to the present invention, a fibrous material made of fibers that enable efficient absorption of substances to be removed in a fluid that is to be treated, and a purification column incorporating the fibers can be provided.

BRIEF DESCRIPTION OF THE DRAWINGS

Fig. 1 depicts a cross-sectional view of a fiber for indicating an inscribed circle and a circumscribed circle.
Fig. 2 depicts a schematic view for explaining each part of a spinneret for producing a fiber with three protrusions extending outwardly from the center of the fiber.
Fig. 3 depicts a cross-sectional view of a fiber with three protrusions extending outwardly from the center of the fiber which is produced using the spinneret as shown in Fig. 2.
Fig. 4 depicts a schematic view of a spinneret for producing a fiber having a cross section mostly resembling a rod shape with round ends.
Fig. 5 depicts a schematic view of a spinneret for producing a fiber having a cross-shaped cross section.
Fig. 6 depicts a schematic view of a spinneret for producing a fiber having a star-shaped cross section.
Fig. 7 depicts a circuit diagram for use in the measurement of the adsorption performance of a column.

MODE FOR CARRYING OUT THE INVENTION

Fibers for use in the present invention consist of fibers called solid fibers, which take a form or configuration of fibers without hollow space. Hollow fibers are concerned about the aforementioned problem and are therefore unsuitable to be used.

Fibers constituting a fibrous material in the present invention are preferably in a monofilament form and the fibrous material may be configured directly from a bundle of solid fiber monofilaments. Plural monofilaments may be intertwined into a single multifilament strand, which is however not preferable because the entangled segments of the monofilaments are hardly in contact with a fluid that is to be treated. The multifilament as used herein includes both multifilaments composed of identical fibers and multifilaments composed of different types of fibers.

In the present invention, a fibrous material that contains plural types of solid fibers having a common cross-sectional shape is used. Blending plural types of fibers in various combinations enables to utilize the features of the respective fibers and to offset the disadvantage of one type of fiber by another type of fiber. One of the desirable effects of fiber blending is as described below: High adsorption performance can be expected by selecting a fiber having a relatively large volume-specific surface area for at least one type of fiber, while use of another fiber having a distinct cross-sectional shape can prevent the above-described fibers having a large volume-specific surface area from overlapping and adhering to each other between neighboring fibers. From such a viewpoint, it will be one important factor to control the surface area increase rates of the solid fibers. As used herein, the surface area increase rate refers to a value given by the following formula (1).

$$\text{Surface area increase rate} = \text{(the circumference of a fiber cross section)} / \text{(the circumference of a circle having the same cross-sectional area as the fiber cross section)} \qquad (1)$$

A measurement method for surface area increase rate is described below. Both ends of a fiber to be measured are fixed with application of a tension of around 0.1 g/mm$^2$ and the fiber is cut at a random position. Then, the cut section is magnified and imaged on a light microscope, the Digital Microscope DG-2 manufactured by Scalar Corporation. At the time of imaging, an image of a scale is also captured under the same magnification. An image analysis software program, "Micro Measure ver. 1.04," manufactured by Scalar Corporation is used to trace the periphery of the cut section of the fiber for measuring the circumference and the cross-sectional area of the fiber cross section. This measurement protocol is repeated for the cross sections at 50 different positions and the obtained values are averaged to give a value of surface area increase rate, rounded to one decimal place.

When, with regard to fibers having a common cross-sectional shape for use in the present invention, the cross sections of two fibers are compared and the comparison identifies one of the fibers, but not the other, as having a protrusion at the periphery of the cross section, those fibers are not considered to have a common form. When the number of protrusions is different between the fibers, those fibers are also not considered to have a common form. When the fibers have the same number of protrusions and, however, have a difference of not less than 30% in the above-described surface area increase rate, those fibers are not considered to have a common form. When a fiber having a round or oval cross section, among fibers having no protrusion, is compared with another fiber similarly having a round or oval cross section and the comparison identifies those fibers as having a difference of not less than 30% in the above-described surface area increase rate or as having a difference of not less than 50% in the below-described modification degree, those fibers are not considered to have a common form. Also, a fiber having a round or oval cross section is not considered to have a common form with a fiber having an angular cross section, while fibers having cross sections with different numbers of angles are not considered to have a common form although each of the fibers has an angular cross section. Also, when fibers have angular cross sections with the same number of angles and, however, have a difference of not less than 30% in the surface area increase rate, those fibers are not considered to have a common form. In reference to the above-described protrusions, the number of protrusions is, for example, 0, 2, 3, 4, 5, and 6 in a fiber cross section having a round or oval shape, having an L shape, having a Y or T shape, having a cross shape, having a star shape, and having an asterisk (*) shape, respectively.

In the present invention, among two or more fibers having a common cross-sectional shape and existing at a composition ratio of not less than 5.0% in the total of fiber blends, a fiber having the highest surface area increase rate is called fiber$_{(max)}$. Such a fiber$_{(max)}$ has a composition ratio of not less than 30.0%, preferably not less than 45.0% and more preferably not less than 55.0%, in the total of fiber blends. When the composition ratio of a fiber$_{(max)}$ is less than 30.0%, improvement of adsorption performance cannot be expected. If plural types of fibers having the highest surface area increase rate as mentioned above are contained, a fiber having a cross-sectional shape with a higher occupancy rate against a circumscribed circle as described below is considered to be a fiber$_{(max)}$ for use in the present invention.

A fiber$_{(max)}$ having a higher surface area increase rate has a larger volume-specific surface area and achieves an improved adsorption performance. Thus, the lower limit of the surface area increase rate of a fiber$_{(max)}$ is not less than 1.20, more preferably not less than 1.35, and particularly preferably not less than 1.45. When the lower limit of the surface area increase rate of a fiber$_{(max)}$ is less than 1.20, the adsorption performance will be insufficient. The upper limit of the surface area increase rate of a fiber$_{(max)}$ is preferably not more than 6.6, further preferably not more than 4.5, and particularly preferably not more than 3.6. When the upper limit of the surface area increase rate of a fiber$_{(max)}$ is within the preferable range, the fiber does not reduce the tensile strength but maintains the spinning stability and easily retains its own form. Moreover, when a spinning solution to be formed into fibers is quickly cooled by air or a liquid, any protrusion on the periphery of the fiber cross section does not excessively hinder the air or liquid flow, which causes difficulty in establishing an irregular fiber form or an irregular microstructure, such as irregularity in pore size.

As used herein, the phrase "a composition ratio in the total of fiber blends" refers to a ratio of the number of fibers having a common cross-sectional shape to the total number of fibers, as given by the following formula (2), in the case where the fibrous materials are cut at an arbitrary position to produce cross sections.

$$\text{Composition ratio in the total of fiber blend materials (\%)} = \text{the number of}$$

$$\text{fibers having a common cross-sectional shape / total number of fibers x 100} \quad (2)$$

Moreover, among fibers having plural types of cross-sectional shapes existing at a composition ratio of not less than 5.0% in the total of fiber blends, a fiber having the lowest surface area increase rate is called fiber$_{(min)}$, which fiber has a surface area increase rate that is reduced by not less than 3.0% as compared to that of a fiber$_{(max)}$. When fibers$_{(min)}$ are contained at a composition ratio of not less than 8.0% in the total of fiber blends, it results in providing an effect to prevent fibers$_{(max)}$ from adhering to each other. The composition ratio of a fiber$_{(min)}$ in the total of fiber blends is preferably not less than 12.0%, more preferably not less than 16.0%, and further preferably not less than 23.0%. The upper limit of the composition ratio of a fiber$_{(min)}$ in the total of fiber blends is preferably not more than 70%, more preferably not more than 60%, further preferably not more than 52%, and particularly preferably not more than 36%. When the upper limit of the composition ratio of a fiber$_{(min)}$ in the total of fiber blends is within the preferable range, it results in formation of a fibrous material having a sufficient amount of surface area and also in preventing fibers$_{(min)}$ from adhering to each other, which serves to maintain the adsorption performance of the fibrous material. If plural types of fibers having the lowest surface area increase rate as mentioned above are contained, a fiber having a cross-sectional shape with a lower occupancy rate against a circumscribed circle as described below is considered to be a fiber$_{(min)}$ for use in the present invention.

In the present invention, it can also be important to control the modification degrees of the solid fibers. The modification degree as used herein refers to a ratio between the diameters of an inscribed circle and a circumscribed circle in the observation of a fiber cross section, namely, a value given by the following formula (3), where $Di$ represents the diameter of an inscribed circle and $Do$ represents the diameter of a circumscribed circle.

$$\text{Modification degree} = Do/Di \qquad\qquad (3)$$

In the present invention, with consideration of a $\text{fiber}_{(min)}$ having a composition ratio of not less than 8.0% in the total of fiber blends, the lower limit of the modification degree of a $\text{fiber}_{(min)}$ is 1.10, more preferably not less than 1.20, further preferably not less than 1.60, and particularly preferably not less than 2.10. When the lower limit of the modification degree of a $\text{fiber}_{(min)}$ is within the preferable range, the whole resulting fibrous material keeps having a bulky effect. On the other hand, the upper limit of the modification degree of a $\text{fiber}_{(min)}$ is not more than 6.00, more preferably not more than 5.00, and particularly preferably not more than 3.80. When the upper limit of the modification degree of a $\text{fiber}_{(min)}$ is within the preferable range, the bulky texture resulting from the $\text{fiber}_{(min)}$ in conjunction with the $\text{fiber}_{(max)}$ is moderate, so that the resulting fibrous material is easy to handle and is easily installed into, for example, a casing. Moreover, the fiber does not reduce the tensile strength but maintains the spinning stability and easily retains its own form. Furthermore, when a spinning solution to be formed into fibers is quickly cooled by air or a liquid, any protrusion on the periphery of the fiber cross section does not excessively hinder the air or liquid flow, which causes difficulty in establishing an irregular fiber form or an irregular microstructure, such as irregularity in pore size.

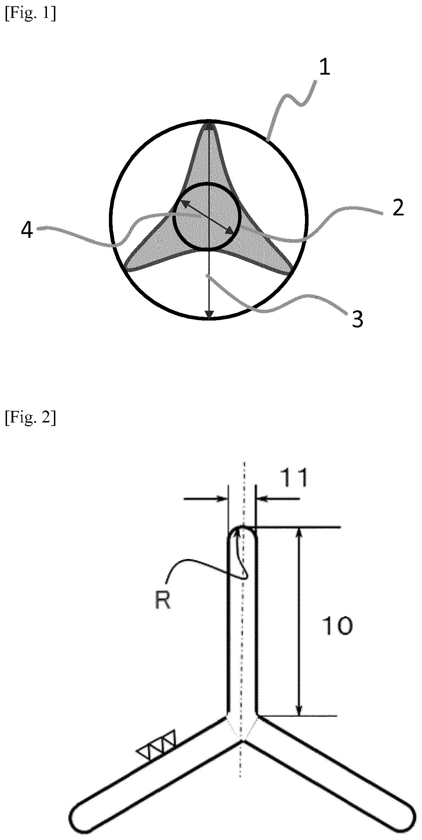

As provided herein, modified cross-sectional shapes may have symmetrical patterns, such as line symmetrical patterns or point symmetrical patterns, or may have asymmetrical patterns, and are preferred to be roughly symmetrical in terms of uniform fabric properties. When a modified cross-sectional shape is determined to have a roughly line symmetrical or point symmetrical pattern, the inscribed circle corresponds to a circle inscribing the outline of a cross section of a fiber and the circumscribed circle corresponds to a circle circumscribing the outline of a cross section of a fiber. Fig. 1 illustrates the circumscribed circle and the inscribed circle of a modified cross-sectional shape in a fiber having three protrusions, and shows the diameters $Do$ and $Di$ of the circles.

Meanwhile, when a modified cross-sectional shape is determined to have neither a line symmetrical pattern nor a point symmetrical pattern, a circle inscribing the outline of a fiber at minimally two points, existing only inside the fiber, and having the largest radius to the extent that the circumference of the inscribed circle does not intersect with the outline of the fiber is considered to be an inscribed circle. On the other hand, a circle circumscribing the outline of a fiber at minimally two points, existing only outside the fiber cross section, and having the smallest radius to the extent that the circumference of a circumscribed circle does not intersect with the outline of the fiber is considered to be a circumscribed circle.

In a measurement method for modification degree, a fiber is cut, magnified and imaged by similar procedures to those for the measurement of surface area increase rate, and the diameters of the circumscribed and inscribed circles of a fiber cross section, $Do$ and $Di,$ are measured using an image analysis software program and then the modification degree is calculated by the above formula (3). This measurement protocol is repeated for the cross sections at 30 different positions and the obtained values are averaged to give a value of modification degree, rounded to one decimal place.

Moreover, it is preferred to control the occupancy rates $Sfo$ against a circumscribed circle in a $\text{fiber}_{(min)}$ and a $\text{fiber}_{(max)}.$ The occupancy rate against a circumscribed circle is given by the following formula (4).

$$\text{Occupancy rate } Sfo \text{ against a circumscribed circle} = \text{(the cross-sectional area}$$
$$\text{of a fiber cross section } Sf) / \text{(the area of the circumscribed circle of the fiber cross}$$

Increase in the $Sfo$ value of a $\text{fiber}_{(max)}$ can lead to an efficient increase of surface area. On the other hand, a smaller $Sfo$ value of a $\text{fiber}_{(min)}$ represents a smaller area occupied by the fiber in a unit cross-sectional area and can lead to acquirement of bulky texture. Thus, the upper limit of the $Sfo$ of a $\text{fiber}_{(min)}$ is not more than 0.90, further preferably not more than 0.70, and particularly preferably not more than 0.50. The lower limit of the $Sfo$ of a $\text{fiber}_{(min)}$ is preferably not less than 0.05, and more preferably not less than 0.15. When the lower limit of the $Sfo$ of a $\text{fiber}_{(min)}$ is within the preferable range, the tensile strength of the fiber is not compromised. Moreover, the $Sfo$ ratio Z obtained by dividing the occupancy rate $Sfo$ against the circumscribed circle of a $\text{fiber}_{(max)}$ by the occupancy rate $Sfo$ against the circumscribed circle of a $\text{fiber}_{(min)}$ is preferably not less than 0.20, more preferably not less than 0.40, further preferably not less than 0.80, and particularly preferably not less than 1.10. When the $Sfo$ ratio Z is within the preferable range, it can lead to an efficient increase of surface area as well as to acquirement of bulky texture, which in turn enables the resulting fibrous material to exhibit excellent adsorption performance.

In a measurement method for occupancy rate against a circumscribed circle, a fiber is cut, magnified and imaged by similar procedures to those for the measurement of modification degree, and the diameter of the circumscribed circle of a fiber cross section, $Do,$ and the cross-sectional area of the fiber cross section, $Sf,$ are measured using an image analysis software program, and a value $So$ is obtained on the basis of the value $Do,$ and then the occupancy rate against a circumscribed circle is calculated by the above formula (4). This measurement protocol is repeated for the cross sections at 30 different positions and the obtained values are averaged to give a value of occupancy rate against the circumscribed circle of the fiber, rounded to one decimal place.

A $fiber_{(max)}$ preferably has a fiber cross section with almost the same equivalent circle diameter as that of a $fiber_{(min)}$. Thus, the upper limit of the fiber diameter ratio Y, which is obtained by dividing the equivalent circle diameter of a $fiber_{(min)}$ by the equivalent circle diameter of a $fiber_{(max)}$ in a fibrous material, is not more than 10.0, further preferably not more than 3.0, and particularly preferably not more than 1.4. Moreover, the lower limit of Y is not less than 0.2, more preferably not less than 0.5, and particularly preferably not less than 0.7. When the fiber diameter ratio Y is within the preferable range, such filaments are not easily breakable by, for example, mechanical impact and, moreover, the whole resulting fibrous material will not decrease its surface area.

Moreover, in reference to the absolute value of equivalent circle diameter, the equivalent circle diameter of a $fiber_{(max)}$ is preferably not less than 10 $\mu$m and not more than 1,000 $\mu$m, and more preferably not less than 20 $\mu$m and not more than 500 $\mu$m. When the equivalent circle diameter of a $fiber_{(max)}$ is within the preferable range, it serves to maintain the mechanical strength of the fiber and to provide sufficient adsorption performance.

In a measurement method for the above-described equivalent circle diameter, both ends of a fiber to be measured are fixed with application of a tension of 0.01 to 0.1 $g/mm^2$ and the filament is cut. Then, the cut section is magnified and imaged on a light microscope, such as, for example, the above-described product of Scalar Corporation. At the time of imaging, an image of a scale is also captured under the same magnification. After digitalizing the image, the above-described image analysis software program manufactured by Scalar Corporation is used to trace the periphery of the cut section of the fiber for calculating the cross-sectional area S, which is used to calculate the individual equivalent aperture diameter by the following formula (5). The average of values measured at 30 different positions is calculated and then rounded to the nearest integer.

$$\text{Equivalent circle diameter of a fiber cross section} = 2 \times (S/\pi)^{1/2} \qquad (5)$$

The raw material of a fiber in the present invention is selected from group consisting of polymethylmethacrylate (hereinafter referred to as PMMA), polyester, polyacrylonitrile (hereinafter referred to as PAN), polysulfone, polyethersulfone, polyarylethersulfone, polypropylene, polystyrene, polycarbonate, cellulose, cellulose triacetate, and ethylene-vinyl alcohol copolymer.

Among those, any raw material which is rather hydrophobic and has a property to enable adsorption of, for example, proteins is preferably contained, and examples of such a raw material include PMMA, PAN and the like. PMMA and PAN are also representative examples of a fiber having a uniform structure in the thickness direction and are prone to provide a homogeneous (porous) structure with a sharp pore size distribution and are therefore preferable. Ester group-containing polymers such as the above-described PMMA are excellent in biocompatibility and are easily modified to express a certain function by controlling the terminal groups and are therefore preferable. In particular, PMMA is an amorphous polymer, which is excellent in molding processability and cost performance and also high in transparency so that, preferably, the internal state of a PMMA fiber is relatively easily observed and the fouling state is easily evaluated.

Moreover, the fiber may carry a negative charge. It is reported that a raw material at least partially containing a functional group carrying a negative charge has increased hydrophilicity and tends to be finely dispersed (in other words, to form many fine pores). Examples of the raw material include raw materials having a substituent as the functional group carrying a negative charge, such as sulfo group, carboxyl group, phosphate group, phosphorous group, ester group, sulfite group, hyposulfite group, sulfide group, phenol group, or hydroxysilyl group. Among those, at least one selected from sulfo group, carboxyl group, and ester group is preferably contained. Examples of a raw material containing sulfo group include, for example, vinylsulfonic acid, acrylsulfonic acid, methacrylsulfonic acid, p-styrenesulfonic acid, 3-methacryloxy-propanesulfonic acid, 3-acryloxy-propanesulfonic acid, and 2-acrylamide-2-methylpropanesulfonic acid; and sodium salts, potassium salts, ammonium salts, pyridine salts, quinoline salts, and tetramethylammonium salts thereof. The quantity of negative charge is preferably not less than 5 $\mu$eq and not more than 30 $\mu$eq per one gram of dry fiber weight. The quantity of negative charge can be measured using, for example, a titration-based method.

Moreover, a $fiber_{(max)}$ according to the present invention is porous and control of the micropore specific surface area of the fiber can increase the adsorption performance on substances to be adsorbed. The lower limit of the micropore specific surface area is not less than 10 $m^2/g$, more preferably not less than 30 $m^2/g$, further preferably not less than 120 $m^2/g$, and particularly preferably not less than 170 $m^2/g$. On the other hand, the upper limit of the micropore specific surface area is preferably not more than 1,000 $m^2/g$, more preferably not more than 800 $m^2/g$, further preferably not more than 650 $m^2/g$, and particularly preferably not more than 500 $m^2/g$. When the upper limit of the micropore specific surface area is within the preferable range, it serves to maintain the mechanical strength. Moreover, although a $fiber_{(min)}$ may have a nonporous structure, an increase of adsorption performance can be expected if a $fiber_{(min)}$ has a micropore specific surface area comparable to that of a $fiber_{(max)}$, as mentioned above.

Micropore specific surface area is obtained through the measurement of the degree of freezing point depression of water in micropores induced by capillary condensation by differential scanning calorimetry (DSC) using a differential scanning calorimeter (DSC), as described in Non-Patent Document 1. The above-described measurement and calculation methods are performed with reference to the description in the aforementioned Non-Patent Document 1.

The porous structure in the solid fibers is an important factor that affects the adsorption rate of substances to be adsorbed. When the average pore radius is small, substances to be adsorbed are hardly introduced into the inside of the pores through the diffusion of the substances and thus the adsorption efficiency is decreased. On the other hand, when the pore radius is too large, substances to be adsorbed are not adsorbed into the void space of the pores and thus the adsorption efficiency is decreased on the contrary. In other words, an optimal pore size is dependent on the size of a substance to be adsorbed and also to be removed, and improper selection of pore size leads to insufficient adsorption of a substance to be adsorbed. Consequently, the average pore radius of the solid fibers is within the range of 1 to 100 nm. When the average pore radius is within this range, the solid fiber can adsorb substances including low molecular-weight substances, proteins, and protein-lipid complexes such as low-density lipoproteins. The average pore radius required to adsorb proteins is not less than 1 nm and not more than 100 nm, and further preferably not less than 5 nm and not more than 50 nm. Similarly to the micropore specific surface area, the average pore radius of a porous fiber is obtained through the measurement of the degree of freezing point depression of water in micropores induced by capillary condensation by differential scanning calorimetry using a differential scanning calorimeter (DSC) as described in Non-Patent Document 1. The above-described measurement and calculation methods are performed with reference to the description in Non-Patent Document 1.

The cross-sectional structure of a filament according to the present invention is not particularly limited, but the fiber preferably has a homogeneous (porous) structure because the presence of a homogeneous porous structure in the thickness direction serves to secure a wider adsorption area. In filaments prepared via, for example, nonsolvent induced phase separation process, a heterogeneous structure with macrovoids is often observed. The macrovoid as used herein refers to a pore with a diameter of not less than 25 $\mu$m. In the present invention, fibers preferably do not have such pores with a diameter of not less than 25 $\mu$m, more preferably not less than 15 $\mu$m, and further preferably not less than 8 $\mu$m. When a fiber does not have voids with a diameter equal to or above a particular size as described above, the volume-specific surface area of the fiber is not reduced, which enables the fiber to keep the physical properties at a required levels. However, when the pore has a shape other than a sphere, such as an ellipsoid, the diameter as used herein refers to the minor diameter of the oval.

As a method of producing the fibrous material of the present invention, both of conventionally known post-spinning fiber blending and spinning-coupled fiber blending techniques can be applied. Examples of the post-spinning fiber blending technique include, but are not limited to, a method in which another type of fibers are fed to a process of fiber blending at the yarn washing or reeling step, a method in which fibers are blended by the air interlacing technique, a method in which fibers are blended by the fiber twisting, yarn doubling, or paralleling technique, a method in which fibers are blended by the combined weaving technique, a method in which fibers are blended by the interknitting technique, and a method in which fibers are blended by dispersing and recovering the fibers in a liquid. Moreover, the composite spinning technique is indicated as an example of the spinning-coupled fiber blending technique. Examples of the composite spinning technique include a method in which a spinneret having multiple orifices is used to simultaneously extrude plural strands of fibers and the resulting fiber strands are reeled, and a method in which an extrusion block containing single-hole spinnerets having different shapes of orifices is used to extrude strands of fibers having different cross-sectional shapes and the resulting fiber strands are reeled. Partly deformed fibers are easily produced by the post-spinning fiber blending technique as compared with the spinning-coupled fiber blending technique and partial deformation may occur on the cross section of a fiber, which causes a problem on the quality stability in the fibrous material. Deformed fibers are hardly produced by the spinning-coupled fiber blending technique and the quality of the resulting fibers can be stable, suggesting that the spinning-coupled fiber blending technique is a preferable technique. Also, it is an advantage of the spinning-coupled fiber blending technique that the combination of fiber forms and the fiber blending ratio can be easily changed by appropriately changing the configuration and arrangement of spinnerets. Additionally, for example, an additive may be supplemented to the extent that the additive will not inhibit the function or effect of the present invention.

Moreover, a fiber$_{(max)}$ and a fiber$_{(min)}$ are preferred to be made from the same raw material. This enables the resulting fibrous materials having common physical and chemical properties, such as having a porous structure, to remove a target substance to be adsorbed in a more effective manner. Moreover, when a multiple spindle spinning machine is used for spinning, an appropriate selection of the configuration of a spinneret, to which a spinning solution is supplied and through which the same spinning solution is extruded, enables fiber blending coincidently with extrusion of the spinning solution through the spinneret, which causes the productivity to be high.

Furthermore, a smaller number of types of fibers having different cross-sectional shapes preferably constitute a fibrous material in terms of productivity and of control of the fibrous material and preferably not more than four, more preferably not more than three, and further preferably only two types of fibers, that is, a fiber$_{(max)}$ and a fiber$_{(min)}$ constitute a fibrous material.

Moreover, the ratio of a fiber(max) to a fiber(min) is preferably 10:1 to 1:2, further preferably 8:1 to 1:1, more preferably 5:1 to 1:1, and particularly preferably 3:1 to 2:1. The fibers may be combined at any ratio between the upper and lower limits of the above-described ratios. This is considered to concomitantly enable the increase of surface area attributed to the fiber(max) and the increase of bulky effect, that is, dispersibility of filaments attributed to the fiber(min).

The configuration or form of a fibrous material to be used in the present invention includes, for example, those of a woven fabric, a knitted fabric, and a nonwoven fabric, and a straight form. Types of woven fabric include, for example, plain weave fabric, twill weave fabric, sateen, mat weave fabric, steep-twill weave fabric, weft-faced weave fabric, and warp-faced weave fabric. Types of knitted fabric include, for example, weft-knitted fabrics such as plain stitch fabric, seed stitch fabric, rib stitch fabric, and interlock knitted fabric. Types of nonwoven fabric include, for example, staple nonwoven fabric, continuous-filament nonwoven fabric, flashspun nonwoven fabric, and melt-blown nonwoven fabric. Among those, knitted fabrics are particularly preferable because fiber flocks are rarely produced from knitted fabrics and the size of openings in a knitted fabric is hardly varied and is easily controlled to be constant. Meanwhile, a fibrous material in a straight form is easily installed into a column in parallel with the axial direction extending between openings on both sides of the column casing and also serves to secure a flow path, in addition to the adsorption material itself, for a fluid that is to be treated, which preferably is advantageous in terms of reduction of flow resistance or of attachment of solutes in a fluid that is to be treated.

Exemplary methods of bundling spun fibers include a method in which the yarn is once wound on a spool and then processed into a form of interest, or a method in which the yarn is directly fed into a processing machine. Examples of the spool include winding plates, angular-shaped spools, and round-shaped spools; winding plates and angular-shaped spools are preferable to produce a bundle of straight fibers.

Although the dispersibility of fibers can be increased by using the fiber blending technique according to the present invention, fibrous materials may be wrapped with, for example, a film, a net, a mesh, or a nonwoven fabric or may be surrounded by a filament such as that called covering filament, for the purpose of promoting easy handling of fibrous materials wound on a spool, that is, the purpose of preventing fibers in a fibrous material from repelling each other because of, for example, static electricity and then from being disorganized.

The fibrous material of the present invention preferably adsorbs $\beta_2$-microglobulin (hereinafter referred to as $\beta_2$-MG) with an adsorption capacity of not less than 0.005 mg/cm$^3$, more preferably not less than 0.014mg/cm$^3$, further preferably not less than 0.020 mg/cm$^3$, and particularly preferably not less than 0.026mg/cm$^3$. When the fibrous material has an adsorption capacity within the preferable range for $\beta_2$-MG and is installed into, for example, a column, the fibrous material exhibits a good adsorption performance, even though installed fibers are small in number, and prevents increase in the volume of the column, and has excellent cost reduction and handling properties. In particular, when blood is a fluid that is to be treated, the fibrous material does not need to increase extracorporeal blood volume and thus does not induce serious adverse drug reactions such as blood pressure drop.

The adsorption performance of a type of filaments can be easily measured by batch, as described below, with focusing on $\beta_2$-MG as an adsorption target, which is the causative protein of a complication of long-term hemodialysis, namely, dialysis-related amyloidosis. First, bovine blood supplemented with sodium azide is adjusted to have a total protein concentration of 6.5 $\pm$ 0.5 g/dL. In addition, bovine plasma stored for up to five days after blood collection is used. Then, $\beta_2$-MG is added to a concentration of 1 mg/L and the resulting mixture is stirred. Furthermore, the length of a fibrous material is adjusted to contain about 100 fibers and to have a volume of 0.0905 cm$^3$ and the resulting fibrous material is introduced into a 15-mL centrifuge tube manufactured by, for example, Greiner Bio-One International GmbH. To the centrifuge tube, 12 mL of the above-described bovine plasma is added, and the bovine plasma in the tube is stirred for one hour at room temperature (20 to 25°C) on, for example, a tilt shaker such as the Wave-SI shaker manufactured by TAITEC Corporation with setting the dial at 38 to cause the platform to tilt up to the largest tilt angle (one round of reciprocal motion for 1.7 seconds). To measure the concentration of $\beta_2$-MG before stirring, $C_1$, (mg/mL) and the concentration of $\beta_2$-MG after stirring, $C_2$, (mg/mL), 1-mL aliquots are sampled and stored in a freezer at -20°C or lower. The concentration of $\beta_2$-MG is measured by the latex coagulation method, and the adsorbed $\beta_2$-MG amount per volume of fiber is calculated based on the following formula (6) and the adsorbed $\beta_2$-MG amount per surface area of fiber is calculated.

$$\text{Adsorbed } \beta_2\text{-MG amount per volume of fiber (mg/cm}^3) = (C_1 - C_2) \times 12 / 0.0905$$

$$(6)$$

wherein $C_1$ represents the concentration(mg/mL) of $\beta_2$-MG before stirring and $C_2$ represents the concentration(mg/mL) of $\beta_2$-MG after stirring.

Geometries as shown in Fig. 2 and Figs. 4 to 7 can be used as the geometry of an orifice in a spinneret to produce a fiber according to the present invention.

The fibrous material according to the present invention can be incorporated into a casing with inlet and outlet ports for a fluid to be treated and then used as a purification column.

Exemplary forms of the casing include prisms, such as square prism or hexagonal prism, and cylinders which have open ends on both sides; among those, cylinders, particularly cylinders having a completely round cross section are preferable because a casing without any corner can prevent blood retention at a corner. Moreover, the presence of open ends on both sides serves to prevent turbulence in the flow of a fluid to be treated, which enables to minimize pressure loss. Moreover, the casing is preferably made from, for example, plastics or metals. Among those, plastics are preferably used in terms of cost, molding processability, weight, blood compatibility and the like. In cases where plastics are used, thermoplastic resins excellent in, for example, mechanical strength and thermostability are used. Specific examples of such thermoplastic resins include polycarbonate-based resins, polyvinyl alcohol-based resins, cellulose-based resins, polyester-based resins, polyarylate-based resins, polyimide-based resins, cyclic polyolefin-based resins, polysulfone-based resins, polyethersulfone-based resins, polyolefin-based resins, polystyrene-based resins, and mixtures thereof. Among those, polypropylene, polystyrene, polycarbonate and derivatives thereof are preferable in terms of molding processability and radiation resistivity, both of which are required for the casing. In particular, resins having excellent transparency, such as polystyrene and polycarbonate, allow the inspection of the inside of the casing, for example, during blood perfusion and thus are suitable for ensuring the safety, while resins having excellent radiation resistivity are suitable for the sterilization of the casing by irradiation. Those resins are produced by ejection molding with a mold or produced by machining of a raw material.

Exemplary methods of covering the ends of a purification column include a method in which the ends of a purification column are covered with mesh sheets, as well as a method in which the ends of a purification column are covered with partition walls made of a resin and then penetrating holes that penetrate the partition walls are provided for the communication between the inside and the outside of the column casing. As used herein, the penetrating holes refer to openings that penetrate the partition walls in the longitudinal direction of fibers. In other words, the penetrating holes refer to openings that locate and penetrate the partition walls formed at both ends of the casing and provide the communication between the inside and the outside of the casing. Among those, the method using mesh sheets has simpler steps to cover the ends of a purification column and also provides more excellent dispersion of a liquid in a column and thus is more preferable than the method in which partition walls are formed at both ends of a casing. Moreover, for the purpose of further increasing the dispersibility of a fluid that is to be treated in a column, a part of the mesh sheet may be replaced with a mesh sheet that cause a larger pressure loss, or with a plate to control the fluid flow, such as those called baffle plate or current plate, may be provided on the mesh sheet.

The form of a fibrous material incorporated into a column is preferred to be a straight form, as mentioned above, and it is preferred to install a bundle of straight fibers into a column substantially in parallel with the axial direction extending between openings on both sides of the column casing. The bundle of straight fibers allows a fluid that is to be treated to flow along the direction of the fibers and thus serves to prevent turbulence in the flow and to easily produce an even distribution of the fluid that is to be treated in a column. Moreover, the bundle of straight fibers enables to reduce the flow resistance and is also advantageous to deal with an increase in pressure loss due to, for example, the attachment of solutes in a fluid that is to be treated. Thus, even if highly viscous blood is the fluid that is to be treated, it is possible to reduce the risk of, for example, blood coagulation in a casing and to prevent problems such as blood remaining in the casing from occurring. The number of straight fibers to be installed into a column is preferably in the range of 1,000 to 500,000.

It is important in the present invention to control the filling ratio of fibers in a casing. The upper limit of the filling ratio is preferably not more than 70%, more preferably not more than 65%, and further preferably not more than 62%. The lower limit of the filling ratio is preferably not less than 20%, more preferably not less than 30%, further preferably not less than 39%, and particularly preferably not less than 47%. The filling ratio refers to a ratio of the volume of fibers (Vf) to the volume of a casing (Vc), which can be determined based on the following formulae (7) to (9), where the volume of a casing (Vc) is calculated from the cross-sectional area and the length of the casing and the volume of fibers (Vf) is calculated from the cross-sectional area of individual fibers and the length of the casing and the number of those fibers.

$$Vc = \text{(the cross-sectional area of the trunk portion of a casing)} \times \text{(the appropriate length)} \tag{7}$$

$$Vf = \text{(the cross-sectional area of a fiber)} \times \text{(the number of fibers)} \times \text{(the appropriate length)} \tag{8}$$

$$\text{Filling ratio} = Vf/Vc \times 100\ (\%) \tag{9}$$

When the filling ratio is within the above-described preferable range, it is easy to prevent filaments from adhering to each other and fibers are smoothly installed into a casing. Meanwhile, with a filling ratio within the above preferable range, it is rare for fibers to be arranged unevenly in a casing, which in turn causes a less uneven distribution of fluid flow in the resulting column and also enables to maintain the adsorption efficiency of the column. Moreover, if blood is the fluid that is to be treated, the resulting column provides an excellent blood reinfusion property and thus rarely causes blood remaining in the column. During blood reinfusion testing, the volume of blood remaining in a column is preferably not more than 5 mL and further preferably not more than 1 mL, though the details of the test will be described below.

When a casing tapers off, the cross-sectional area at the middle position of the casing is defined as the cross-sectional area of the trunk portion of the casing. Moreover, the Vc as used herein is intended not to include the volume of members that do not include fibers, particularly the volume of members that work as an outlet or inlet port for discharge or entry of a fluid to be treated and usually are not filled directly with fibers, such as those called headers or header caps. When adhesion of fibers to each other is prevented in a casing by using, for example, spacer fibers, the Vf is intended to also include the volume of the spacer fibers. The appropriate length of fibers refers to a length obtained by subtracting the length of partition walls formed at both ends of a casing from the length of the casing. The upper limit of the appropriate length of such fibers can be varied depending on their use and is preferably not more than 5,000 mm, more preferably not more than 500 mm, and particularly preferably not more than 210 mm in terms of increase in pressure loss due to bending of fibers or installation of fibers into a column. Moreover, when the appropriate length is too short, fibers should be prepared to have a defined length by, for example, cutting off the excess length of the fibers that are exposed from a column and thus the amount of fibers to be discarded is increased so that the productivity is reduced. Therefore, a too short appropriate length of fibers is not preferable. Furthermore, a too short appropriate length of fibers causes, for example, difficulty in handling the fibrous materials. Thus, the lower limit of the appropriate length of fibers is preferably not less than 5 mm, more preferably not less than 20 mm, and particularly preferably not less than 30 mm. In a measurement method for the appropriate length of fibers, a crinkled fiber, such as a crimped fiber, is straightened by pulling both ends of the fiber in opposite directions and then the length of the fiber is measured. Specifically, one end of a fiber withdrawn from a column is fixed with an adhesive tape and the other end is attached to a sinker of about 5 g per $mm^2$ of fiber cross-sectional area to allow the fiber to hang vertically and the length of the straightened fiber is promptly measured. This measurement protocol is repeated for 100 fibers randomly selected from fibers in a column and the average length of the 100 fibers is calculated in mm and then rounded to the nearest integer.

Moreover, the inventors found that equivalent diameter of flow cross-section was important in the present invention for increasing chances of substances to be adsorbed to contact with solid fibers while a fluid that is to be treated passes through a column. As used herein, the flow path refers to a space between individual solid fibers arranged substantially in parallel with the longitudinal direction of a column, through which a fluid that is to be treated can pass in the column, whereas the cross-sectional area of flow refers to a cross section perpendicular to the axial direction extending between openings on both sides of the casing. Moreover, the equivalent diameter refers to the diameter of a cross-sectional area which is assumed to be round and particularly refers to the size of a flow path obtained by the following formula (10). The upper limit of the equivalent diameter of flow cross-section is not more than 250 $\mu$m, more preferably not more than 200 $\mu$m, and further preferably not more than 150 $\mu$m. The lower limit of the equivalent diameter of flow cross-section is not less than 10 $\mu$m, and more preferably not less than 30 $\mu$m. When the equivalent diameter of flow cross-section is within the above-described preferable range, it enables to sufficiently secure chances of substances to be adsorbed to contact with the surface of solid fibers and meanwhile prevents an excessive increase of pressure loss in the column, which results in less activation of blood.

As described above, the equivalent diameter of flow cross-section ($D_p$) in a fibrous material composed of only one type of solid fibers having a common cross-sectional shape is obtained based on the following formula (10).

$$D_p = 4 \times ((D_{case}/2)^2 - (D_{fiber}/2)^2 \times N) / (D_{case} + D_{fiber} \times N) \tag{10}$$

In the above-described formula (10), $D_{case}$ represents the inner diameter of a column casing, and $D_{fiber}$ represents the equivalent circle diameter of a fiber, and N represents the number of fibers.

In the case of a fibrous material that contains plural types of solid fibers having a common cross-sectional shape as in the present invention, the equivalent diameter of flow cross-section ($D_p$) is obtained based on the following formula (11).

$$D_p = 4 \times ((D_{case}/2)^2 - (D_{fiber\text{-}max}/2)^2 \times N_{max} - (D_{fiber\text{-}min}/2)^2 \times N_{min}) / (D_{case} + D_{fiber\text{-}max} \times SA_{max} \times N_{max} + D_{fiber\text{-}min} \times SA_{min} \times N_{min}) \qquad (11)$$

In the above-described formula (11), $D_{case}$ represents the inner diameter of a column casing, and $D_{fiber\text{-}max}$ represents the equivalent circle diameter of a fiber$_{(max)}$, and $D_{fiber\text{-}min}$ represents the equivalent circle diameter of a fiber$_{(min)}$, and Nmax represents the number of fibers$_{(max)}$, and $N_{min}$ represents the number of fibers$_{(min)}$, and $SA_{max}$ represents the surface area increase rate of a fiber$_{(max)}$, and $SA_{min}$ represents the surface area increase rate of a fiber$_{(min)}$.

In the present invention, fibers$_{(max)}$ are porous and therefore proteins are incorporated into and adsorbed by the fibers. Thus, those fibers are required to have a fiber form and a porous structure that allow proteins to easily move into the inside of the fibers. Furthermore, the inventors found that, in the present invention, an increase of pressure loss in a column promotes movement of proteins into the inside of the solid filbers. On the other hand, when the amount of pressure loss is too large, it results in activation of blood. In other words, the amount of pressure loss caused by allowing bovine blood to flow through a column at a flow rate of 200 mL/min is not less than 0.5 kPa, preferably not less than 1.5 kPa, more preferably not less than 3.0 kPa, whereas the upper limit of the amount of pressure loss is not more than 30 kPa. and particularly preferably not more than 20 kPa. The pressure loss can be controlled by adjusting, for example, the filling ratio of solid fibers in a column, the inner diameter of the casing, the diameter of solid fibers, and the number of solid fibers. A measurement method for pressure loss will be described below. Such a fibrous material and a purification column incorporating the same have a variety of uses and can be used for applications such as water treatment, purification, and medical treatment. In medical applications among those, a purification column of the present invention can be used in either of the following processing methods for blood purification: a method in which a column is directly perfused with whole blood, and a method in which plasma or serum is once separated from blood and then a column is perfused with the separated plasma or serum.

When the adsorption performance of a column of the present invention is evaluated by using $\beta_2$-MG for example, circulation of blood in the column having 3 m$^2$ of blood-contacting fiber surface area at a flow rate 200 mL/min for one hour results in a clearance rate of preferably not less than 40 mL/min, more preferably not less than 50 mL/min, and further preferably not less than 60 mL/min. Moreover, the volume of blood (the case part) in the column having a blood-contacting fiber surface area of not less than 3 m$^2$ is preferably not more than 170 mL, and further preferably not more than 130 mL. When it is difficult to produce a column having a blood-contacting surface area of 3 m$^2$, the clearance measured in a column having a volume of 1 m$^2$ to 7 m$^2$ may be converted to that in a column having 3 m$^2$ on the basis of the overall mass transfer coefficient Ko as described below. A measurement method for clearance will be described below in the section "Measurement of the adsorption performance of a column".

Moreover, when a purification column of the present invention is used as a medical appliance for extracorporeal circulation, a preferable method comprises incorporating the column into an extracorporeal circulation circuit to perform an adsorptive removal operation in an on-line mode in terms of the throughput achieved by a single process or of operational convenience. In this case, the purification column of the present invention may be used either singly or connected in series with an artificial kidney during, for example, hemodialysis. Use of such a procedure enables removal of substances that are only insufficiently removed by an artificial kidney along with hemodialysis. In particular, use of a purification column according to the present invention enables adsorptive removal of high molecular-weight substances that are hardly removed by artificial kidneys, by which the deficit in the function of artificial kidneys can be covered.

When the purification column is used together with an artificial kidney, the purification column may be connected ahead of or next to the artificial kidney in a circuit. An advantage of connecting the purification column ahead of an artificial kidney is that the configuration prevents the purification column from being affected by hemodialysis on the artificial kidney and thus the purification column easily exhibits its intrinsic performance. On the other hand, an advantage of connecting the purification column next to an artificial kidney is that a pool of blood once treated by the artificial kidney to remove water is further processed on the purification column, whereby the concentrations of solutes in the pool of blood is increased and thus an enhanced efficiency in adsorptive removal can be expected.

Moreover, a fibrous material modified in advance to hold, for example, an agent in its micropores can allow the fibrous material to have functions, such as controlled release of the agent and the like, as well as the adsorption function. In an example, when the fibrous material is used as a medical appliance, the fibrous material modified in advance to hold an anticoagulant can increase the antithrombogenicity.

In another example, when the fibrous material is used in a medical application, the fibrous material modified to contain fibers$_{(max)}$ and/or fibers$_{(min)}$ with protrusions can actively remove, for example, an excess amount of activated leukocytes in blood. The mechanism of the removal is uncertain, but it is believed to be resulting from recognition of the protrusions as a foreign substance and the resulting exertion of phagocytic activity by activated leukocytes.

Exemplary production processes for a fibrous material according to the present invention and a purification column incorporating the same will be described below.

<Production of a fibrous material>

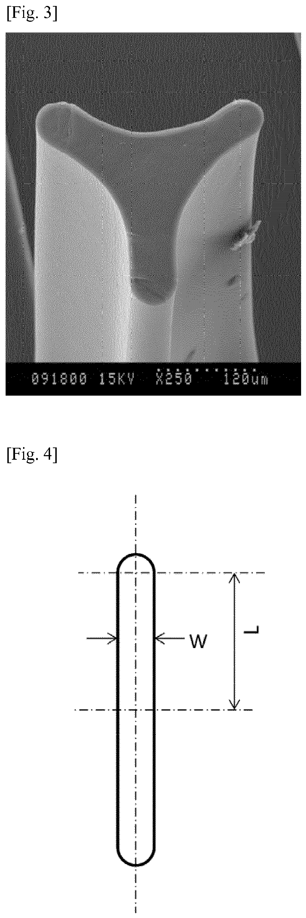

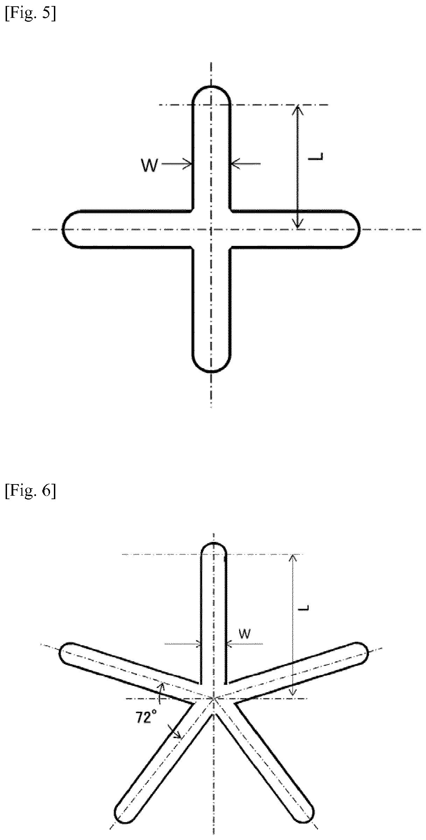

A spinning solution composed of a polymer dissolved in a solvent is prepared. When the spinning solution has a lower concentration (the concentration of substances excluding the solvent in the spinning solution), fibers having micropores with larger pore sizes can be produced. Thus, an appropriate selection of spinning solution concentration enables control of pore size. In addition, use of a polymer containing a negatively charged group also enables control of pore size. From such a viewpoint, in the present invention, the solution concentration is preferably not more than 30% by mass, more preferably not more than 27% by mass, and further preferably not more than 24% by mass. Such a spinning solution can be extruded through a spinneret and allowed to pass through a certain distance of space in a dry air chamber and then delivered into a coagulating bath containing a poor solvent such as water or containing a nonsolvent for coagulation of the polymer to obtain fibers. As the spinneret, a spinneret having an orifice of a modified cross-sectional shape as shown in, for example, Fig. 5 (the width of a slit W = 0.10 mm, the length of a slit L = 1.0 mm) and Fig. 4 (the width of a slit W = 0.10 mm, the length of a slit L = 1.0 mm) is used. Combinational use of those two types of spinnerets at a number ratio of, for example, 1:1 enables to blend different types of fibers during fiber spinning. In the cross-sectional shapes of the orifices, the sagitta of the arc R is preferably equal to one half of the width of the slit. An alternate arrangement of the spinnerets having the orifices with the different cross-sectional shapes for the production of fibrous materials can reduce the frequency of formation of an assembly composed of modified cross-section fibers having a common cross-sectional shape. The extruded spinning solution is allowed to pass through a certain distance of space in a dry air chamber and then delivered into a coagulating bath containing a poor solvent such as water or containing a nonsolvent. From the above-described viewpoint, the lower limit of the transition (retention) time of fibers in the dry air chamber is as described above. Moreover, when the structure of extruded fibers is promptly fixed in the dry air chamber by reducing the temperature of the extruded fibers to the gelation or coagulation temperature, cold air is blown over the extruded fibers in the dry air chamber to promote gelation. Moreover, a detailed mechanism is uncertain, but the rate of pore formation on the surface of fibers or the size of pores near the periphery of fibers can be increased by increasing the cold-air blowing rate and thus increasing the cooling efficiency.

As described above, a spinning solution extruded through a spinneret is coagulated in a coagulating bath, which is usually composed of a coagulating agent such as water or an alcohol, or a mixture of a coagulating agent and a solvent that is a constituent of the spinning solution. It is usual that water is often used. Moreover, the size of pores can be changed by controlling the temperature of a coagulating bath. Since the pore size can be affected by, for example, the type of a spinning solution, the temperature of a coagulating bath should also be appropriately selected. In general, the size of pores can be increased by increasing the temperature of a coagulating bath. The exact mechanism of increase in pore size is uncertain, but it is believed to be resulting from completion of coagulation before shrinkage of the inner regions of fibers, which reflects fast solvent removal caused by bathing at a high temperature in a competitive reaction between solvent removal from a spinning solution and solidification shrinkage. However, when the temperature of a coagulating bath is too high, the pore size is increased too much, which is believed to cause effects, such as decrease in micropore specific surface area, decrease in tensile strength, and increase in non-specific adsorption. Thus, the temperature of a coagulating bath for, for example, fibers containing PMMA is preferably not higher than 90°C, more preferably not higher than 75°C, and particularly preferably not higher than 65°C. On the other hand, when the temperature of a coagulating bath is too low, the pore size is decreased, which causes difficulty in allowing proteins to diffuse into the inside of pores. Thus, the lower limit is preferably not lower than 12°C and more preferably not lower than 20°C.

Next, the extruded fibers experience a step of washing off the solvent on the coagulated fibers. The washing means is not particularly limited, but a method of washing fibers sequentially in multiple containers full of water (referred to as water washing baths) is preferably used. The temperature of water in the water washing baths may be determined depending on the properties of the polymer which is a constituent of the fibers. For example, the temperature is selected from 30 to 50°C in the case of fibers containing PMMA.

Moreover, the water bath step may be followed by a step of applying a moisturizing ingredient to the fibers in order to maintain the size of pores. The moisturizing ingredient as used herein refers to an ingredient capable of maintaining the moisture of fibers, or an ingredient capable of preventing the moisture of fibers from being reduced in the air. Representative examples of the moisturizing ingredient include glycerol and an aqueous solution thereof.

The fibers which have been washed or applied with a moisturizing ingredient may experience a step of immersing the fibers in a container full of a heated aqueous solution of a moisturizing ingredient (referred to as heat treatment bath) in order to increase the dimensional stability of the highly shrinkable fibers. The heat treatment bath is filled with a heated aqueous solution of a moisturizing ingredient, and fibers treated in the heat treatment bath shrink under the influence of the thermal action and become less shrinkable in the later steps, whereby the fibers can stabilize their own structure. The temperature for the thermal treatment is varied depending on the raw material of fibers, and is preferably not lower than 50°C and more preferably not lower than 80°C in the case of fibers containing PMMA. Moreover, the temperature is preferably not higher than 95°C and more preferably not higher than 87°C.

The yarn is wound on a spool, as mentioned above, whereby a fibrous material can be produced. A fibrous material in a straight form without any disorder of fibers is prepared by using an angular-shaped spool, such as a spool with a hexagonal cross section.

<Production of a purification column>

An example of producing a purification column with the obtained fibrous material will be described below.

First, the fibrous material is cut into pieces of a required length and the cut pieces of the fibrous material are installed into a plastic casing for a purification column, which corresponds to the trunk portion of the purification column, straight in the axial direction of the casing. Then, both ends of the fibrous material are cut with, for example, a cutter knife such that the fibrous material is accommodated in the casing, and the outlet and inlet openings for a fluid to be treated at the ends on both sides of the column are covered with mesh filters cut into a shape having the same diameter as the inner diameter of the column. Finally, so-called header caps with inlet and outlet ports for a fluid to be treated can be attached on both ends of the casing to obtain a purification column.

Moreover, when the purification column is used as, for example, a medical appliance, namely, as a medical adsorption column, the purification column is preferred to be disinfected or sterilized before use. Examples of the disinfection or sterilization method include various disinfection or sterilization methods, such as high-pressure steam sterilization, gamma-ray sterilization, electron-beam sterilization, ethylene oxide gas sterilization, disinfectant-based sterilization, and ultraviolet-ray sterilization. Among those methods, gamma-ray sterilization, electron-beam sterilization, high-pressure steam sterilization, and ethylene oxide gas sterilization have less influence on sterilization efficiency and on materials and are therefore preferable.

With respect to every item defined by a stated numerical range in this specification, the numerical range may be defined by any combination of an upper limit selected from a stated upper limit value, a stated preferable upper limit value and a stated more preferable upper limit value of said item and a lower limit value selected from a stated lower limit value, a stated preferable lower limit value and a stated more preferable lower limit value of said item.

EXAMPLES

Example 1

<Production of a fibrous material>

In 376 parts by mass of dimethyl sulfoxide, 31.7 parts by mass of a syndiotactic PMMA (hereinafter referred to as syn-PMMA) polymer having a weight average molecular weight of 400,000, 31.7 parts by mass of a syn-PMMA polymer having a weight average molecular weight of 1400,000, 16.7 parts by mass of a isotactic PMMA (hereinafter referred to as iso-PMMA) polymer having a weight average molecular weight of 500,000, and 20 parts by mass of a PMMA-based copolymer having a molecular weight of 300,000 that contains sodium p-styrenesulfonate at a ratio of 1.5 mol% were mixed and the resulting mixture was stirred at 110°C for 8 hours to prepare a spinning solution. The viscosity of the obtained spinning solution at 110°C was 1,240 poise. A spinneret having an orifice of a shape as shown in Fig. 5 and with dimensions as indicated in Table 1 and another spinneret having an orifice of a round shape with a diameter (ø) of 0.3 were combined at a ratio of 2:1 and the obtained spinning solution was extruded through the resulting spinneret block kept at 92°C into air space by allowing the spinning solution to pass through the respective spinnerets at a rate of 1.1 g/min, and the extruded spinning solution traveled 500 mm in the air space and then was delivered into a coagulating bath, and the spinning solution that had passed through the bath gave solid fibers. Water was used in the coagulating bath and the water temperature (coagulating bath temperature) was 42°C. The fibers produced using the respective spinnerets were washed in water and subsequently delivered into a container full of an aqueous solution containing glycerol at 70% by mass as a moisturizing agent and then allowed to pass through a heat treatment bath at 84°C for the purpose of removing an excess amount of glycerol. Then, 120,000 fibers were wound on a spool with a hexagonal cross section at a rate of 16 m/min to give a fibrous material, which is a fiber blend bundle composed of fibers$_{(max)}$ and fibers$_{(min)}$.

The surface area increase rate, the modification degree, the occupancy rate against a circumscribed circle, the micropore specific surface area, the equivalent circle diameter and the like were measured in the obtained filament by the above-described methods. The results are presented in Tables 1 to 4.

<Production of a column>

The obtained fibrous material was incorporated in a straight form into a cylindrical casing made of polycarbonate with an inner diameter of 10 mm and an axial length of 18 mm to give a filling ratio of fibers at 57%. Then, the outlet and inlet openings for a fluid to be treated at the ends on both sides of the casing were covered with mesh filters with an opening equivalent diameter of 84 $\mu$m and an aperture ratio of 36%, which mesh filters were cut into a shape having the same diameter as the inner diameter of the casing. Finally, caps with inlet and outlet ports for a fluid to be treated, which are called headers, were attached on the ends of the casing.

<Measurement of surface area increase rate, modification degree, occupancy rate against a circumscribed circle, and equivalent circle diameter of a fiber cross section>

As mentioned above, a fiber to be measured was cut at a random position and the cut section was magnified and imaged on the Digital Microscope DG-2 manufactured by Scalar Corporation. At the time of imaging, an image analysis software program, "Micro Measure ver. 1.04", manufactured by Scalar Corporation was also used to capture an image of a scale under the same magnification. The aforementioned measurement and calculation were performed to obtain the surface area increase rate and the modification degree.

<Measurement of the adsorption performance of a fibrous material>

Up to five days after blood collection, bovine blood supplemented with sodium azide was adjusted to have a hematocrit of 30 $\pm$ 3% and a total protein concentration of 6.5 $\pm$ 0.5 g/dL. Then, $\beta_2$-MG was added to a concentration of 1 mg/L and the resulting mixture was stirred. Furthermore, the length of a fibrous material was adjusted to contain about 100 fibers and to have a volume of 0.0905 cm$^3$ and the resulting fibrous material was introduced into a 15-mL centrifuge tube manufactured by, for example, Greiner Bio-One International GmbH. To the centrifuge tube, 12 mL of the above-described bovine plasma was added, and the bovine plasma in the tube was stirred for one hour at room temperature (20 to 25°C) on, for example, a tilt shaker such as the Wave-SI shaker manufactured by TAITEC Corporation with setting the dial at 38 to cause the platform to tilt up to the largest tilt angle (one round of reciprocal motion for 1.7 seconds). To measure the concentration of $\beta_2$-MG before stirring, $C_1$, (mg/mL) and the concentration of $\beta_2$-MG after stirring, $C_2$, (mg/mL), 1-mL aliquots were sampled and stored in a freezer at - 20°C or lower. The concentration of $\beta_2$-MG was measured by the latex coagulation method, and the adsorbed amount per volume of fiber was calculated based on the following formula (12).

$$\text{Adsorbed amount per volume of fiber (mg/cm}^3) = (C_1 - C_2) \times 12 / 0.0905$$

$$(12)$$

<Measurement of the adsorption performance of a column>

The clearance of $\beta_2$-MG was measured to evaluate the adsorption performance of a column. Bovine blood supplemented with sodium azide was centrifuged to obtain plasma. The plasma was adjusted to have a total protein concentration of 6.5 $\pm$ 0.5 g/dL. The bovine plasma stored for up to five days after blood collection was used. Then, $\beta_2$-MG was added to a concentration of 1 mg/L in the bovine plasma and the resulting mixture was stirred. The bovine plasma was divided two portions: one having a volume of 35 mL for circulation and the other having a volume of 40 mL for clearance measurement.

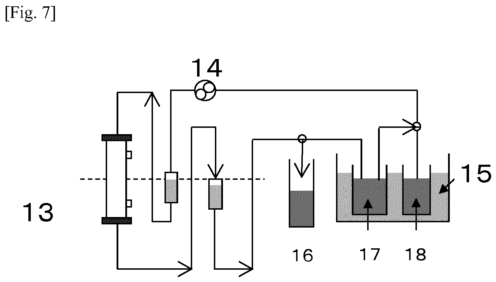

A circuit was prepared as shown in Fig. 7. In the circuit, a port for introducing a fluid to be treated was designated as Bi and a port for discharging a fluid passing through a purification column was designated as Bo.

The Bi was placed into a beaker for a circulating fluid, which contained 35 mL (at 37°C) of the above adjusted bovine plasma, and pumping at a flow rate of 3.5 mL/min was started. Immediately after discarding a volume of a fluid that was discharged from the Bo for the first 90 seconds, the Bo was placed into the beaker for a circulating fluid to establish the circulation. The circulation of the fluid was continued for one hour and then the pump was stopped.

Next, the Bi was placed into the bovine plasma for clearance measurement as adjusted and the Bo was placed into a beaker for waste.

Two minutes after starting pumping at a flow rate of 3.5 mL/min, an aliquot of 1 mL was withdrawn from the bovine plasma (at 37°C) for clearance measurement and designated as Bi liquid. Four minutes and thirty seconds after starting pumping, 1 mL of a sample discharged from the Bo was collected and designated as Bo liquid. These samples were stored in a freezer at -20°C or lower.

The clearance was calculated from the concentration of $\beta_2$-MG in each of the liquids based on the following formula (13). Since measurement values could vary depending on each bovine blood lot, the same lot of bovine plasma was used in all of

Examples and Comparative Examples.

$$Co \text{ (mL/min)} = (CBi - CBo) \times Q_B / CBi \qquad (13)$$

In the above formula (13), $C_o$ represents the clearance of $\beta_2$-MG (mL/min), CBi represents the concentration of $\beta_2$-MG in the Bi liquid, CBo represents the concentration of $\beta_2$-MG in the Bo liquid, and $Q_B$ represents the flow rate (mL/min) for pumping from the Bi. Moreover, the adsorption performance per surface area was obtained by calculating Ko based on the following formula (14).

$$Ko \text{ (cm/min)} = Q_B/A \times \ln[Q_B/(Q_B - Co)] \qquad (14)$$

In the above formula (14), Ko represents the overall mass transfer coefficient of $\beta_2$-MG (cm/min) and A represents the total fiber surface area (cm$^2$) of a fibrous material.

The results are presented in Table 4.

Examples 2 to 5

Fibrous materials and columns were produced under the same conditions as in Example 1, except that a spinneret having an orifice with a shape and dimensions as indicated in Table 1 and another spinneret having an orifice with a shape and dimensions as indicated in Table 2 were combined at a ratio of 2:1. The results are presented in Tables 3 and 4.

Example 6

A fibrous material and a column were produced under the same conditions as in Example 1, except that a spinneret having an orifice with a shape and dimensions as indicated in Table 1 and another spinneret having an orifice with a shape and dimensions as indicated in Table 2 were combined at a ratio of 1:2. The results are presented in Tables 3 and 4.

Example 7

A fibrous material and a column were produced under the same conditions as in Example 1, except that a spinneret having an orifice with a shape and dimensions as indicated in Table 1 and another spinneret having an orifice with a shape and dimensions as indicated in Table 2 were combined at a ratio of 1:1. The results are presented in Tables 3 and 4.

Example 8

A fibrous material and a column were produced under the same conditions as in Example 1, except that a spinneret having an orifice with a shape and dimensions as indicated in Table 1 and another spinneret having an orifice with a shape and dimensions as indicated in Table 2 were combined at a ratio of 2.5:1 (= 5:2). The results are presented in Tables 3 and 4.

Example 9

A fibrous material and a column were produced under the same conditions as in Example 1, except that a spinneret having an orifice with a shape and dimensions as indicated in Table 1 and another spinneret having an orifice with a shape and dimensions as indicated in Table 2 were combined at a ratio of 3:1. The results are presented in Tables 3 and 4.

Example 10

A fibrous material and a column were produced under the same conditions as in Example 1, except that a spinneret having an orifice with a shape and dimensions as indicated in Table 1 and another spinneret having an orifice with a shape and dimensions as indicated in Table 2 were combined at a ratio of 5:1. The results are presented in Tables 3 and 4.

Example 11

A fibrous material and a column were produced under the same conditions as in Example 1, except that a spinneret having an orifice with a shape and dimensions as indicated in Table 1 and another spinneret having an orifice with a shape and dimensions as indicated in Table 2 were combined at a ratio of 8:1. The results are presented in Tables 3 and 4.

Example 12

A fibrous material and a column were produced under the same conditions as in Example 1, except that a spinneret having an orifice with a shape and dimensions as indicated in Table 1 and another spinneret having an orifice with a shape and dimensions as indicated in Table 2 were combined at a ratio of 10:1. The results are presented in Tables 3 and 4.

Example 13

A fibrous material and a column were produced under the same conditions as in Example 1, except that a spinneret having an orifice with a shape and dimensions as indicated in Table 1 and another spinneret having an orifice with a shape and dimensions as indicated in Table 2 were combined at a ratio of 2:1 and the extrusion rates were 1.1 g/min for the former spinneret and 0.41 g/min for the latter spinneret. The results are presented in Tables 3 and 4.

Example 14

A fibrous material and a column were produced under the same conditions as in Example 1, except that a spinneret having an orifice with a shape and dimensions as indicated in Table 1 and another spinneret having an orifice with a shape and dimensions as indicated in Table 2 were combined at a ratio of 2:1 and the extrusion rates were 1.1 g/min for the former spinneret and 0.72 g/min for the latter spinneret. The results are presented in Tables 3 and 4.

Example 15

A fibrous material and a column were produced under the same conditions as in Example 1, except that a spinneret having an orifice with dimensions as indicated in Table 1 and another spinneret having an orifice with dimensions as indicated in Table 2 were combined at a ratio of 2:1 and the extrusion rates were 1.1 g/min for the former spinneret and 1.30 g/min for the latter spinneret. The results are presented in Tables 3 and 4.

Example 16

A fibrous material and a column were produced under the same conditions as in Example 1, except that a spinneret having an orifice with a shape and dimensions as indicated in Table 1 and another spinneret having an orifice with a shape and dimensions as indicated in Table 2 were combined at a ratio of 2:1 and the extrusion rates were 1.1 g/min for the former spinneret and 1.90 g/min for the latter spinneret. The results are presented in Tables 3 and 4.

Example 17

A fibrous material and a column were produced under the same conditions as in Example 1, except that a spinneret having an orifice with a shape and dimensions as indicated in Table 1 and another spinneret having an orifice with a shape and dimensions as indicated in Table 2 were combined at a ratio of 2:1 and the extrusion rates were 1.1 g/min for the former spinneret and 5.00 g/min for the latter spinneret. The results are presented in Tables 3 and 4.

Examples 18 to 21

Fibrous materials and columns were produced under the same conditions as in Example 1, except that a spinneret having an orifice with a shape and dimensions as indicated in Table 1 and another spinneret having an orifice with a shape and dimensions as indicated in Table 2 were combined at a ratio of 2:1. The results are presented in Tables 3 and 4.

Comparative Example 1

A fibrous material and a column were produced under the same conditions as in Example 1, except that only a spinneret having an orifice with a shape and dimensions as indicated in Table 1 was installed. The results are presented in Tables 3 and 4.

Comparative Example 2

A fibrous material and a column were produced under the same conditions as in Example 1, except that only a spinneret having an orifice with a shape and dimensions as indicated in Table 1 was installed. The results are presented in Tables 3 and 4.

Comparative Example 3

A fibrous material and a column were produced under the same conditions as in Example 1, except that only a spinneret having an orifice of a round shape was installed. The results are presented in Tables 3 and 4.

Comparative Example 4

A fibrous material and a column were produced under the same conditions as in Example 1, except that a spinneret having an orifice with a shape and dimensions as indicated in Table 1 and another spinneret having an orifice with a shape and dimensions as indicated in Table 2 were combined at a ratio of 1:5. The results are presented in Tables 3 and 4.

Comparative Example 5

A fibrous material and a column were produced under the same conditions as in Example 1, except that a spinneret having an orifice with a shape and dimensions as indicated in Table 1 and another spinneret having an orifice with a shape and dimensions as indicated in Table 2 were combined at a ratio of 24:1. The results are presented in Tables 3 and 4.

Comparative Example 6

A fibrous material and a column were produced under the same conditions as in Example 1, except that a commercial nylon fishing line (size, 0.5; manufactured by Fujinoline Corporation) was used as an adsorption material. The results are presented in Tables 3 and 4.

[Table 1]

| | Shape & Size of Spinneret | | | | Extrusion Rate (cc/min) | Fiber (max) | | | | | | | |
| | Shape of Spinneret (Figure No.) | W (mm) | L (mm) | L/W | | Equivalent Circle Diameter (μm) | Protrusion Numbers at Fiber Cross section (pieces) | Fiber Cross-sectional Shape | Surface Area Increase Rate | Modification Degree (Outer/Inner) | Occupancy Rate against a Circumscribed Circle | Specific Surface Area (m²/g) | Numbers of Fibers (strands) |
|---|---|---|---|---|---|---|---|---|---|---|---|---|---|
| Example 1 | Figure 5 | 0.10 | 1.0 | 10.0 | 0.6 | 120 | 4 | Cross | 1.55 | 2.3 | 0.47 | 311 | 2,600 |
| Example 2 | Figure 5 | 0.10 | 1.0 | 10.0 | 0.6 | 120 | 4 | Cross | 1.55 | 2.3 | 0.47 | 311 | 2.600 |
| Example 3 | Figure 5 | 0.10 | 1.0 | 10.0 | 0.6 | 120 | 4 | Cross | 1.55 | 2.3 | 0.47 | 311 | 2,600 |
| Example 4 | Figure 5 | 0.10 | 1.0 | 10.0 | 0.6 | 120 | 4 | Cross | 1.55 | 2.3 | 0.47 | 311 | 2,600 |
| Example 5 | Figure 5 | 0.10 | 1.0 | 10.0 | 0.6 | 120 | 4 | Cross | 1.55 | 2.3 | 0.47 | 311 | 2,600 |
| Comparative Example 1 | Figure 5 | 0.10 | 1.0 | 10.0 | 0.6 | 120 | 4 | Cross | 1.55 | 2.3 | 0.47 | 311 | 3,900 |
| Comparative Example 2 | Figure 4 | 0.10 | 1.0 | 10.0 | 0.6 | 120 | 0 | Oval | 1.20 | 2.6 | 0.42 | 299 | 3,900 |
| Comparative Example 3 | Circle | - | - | - | 0.6 | 120 | 0 | Circle | 1.00 | 1.0 | 1.0 | 305 | 3,900 |
| Comparative Example 4 | Figure 5 | 0.10 | 1.0 | 10.0 | 0.6 | 120 | 4 | Cross | 1.55 | 2.3 | 0.47 | 311 | 3,250 |
| Example 6 | Figure 5 | 0.10 | 1.0 | 10.0 | 0.6 | 120 | 4 | Cross | 1.55 | 2.3 | 0.47 | 311 | 2,600 |
| Example 7 | Figure 5 | 0.10 | 1.0 | 10.0 | 0.6 | 120 | 4 | Cross | 1.55 | 2.3 | 0.47 | 311 | 1,950 |
| Example 8 | Figure 5 | 0.10 | 1.0 | 10.0 | 0.6 | 120 | 4 | Cross | 1.55 | 2.3 | 0.47 | 311 | 2,790 |
| Example 9 | Figure 5 | 0.10 | 1.0 | 10.0 | 0.6 | 120 | 4 | Cross | 1.55 | 2.3 | 0.47 | 311 | 2.925 |
| Example 10 | Figure 5 | 0.10 | 1.0 | 10.0 | 0.6 | 120 | 4 | Cross | 1.55 | 2.3 | 0.47 | 311 | 3,250 |
| Example 11 | Figure 5 | 0.10 | 1.0 | 10.0 | 0.6 | 120 | 4 | Cross | 1.55 | 2.3 | 0.47 | 311 | 3,470 |
| Example 12 | Figure 5 | 0.10 | 1.0 | 10.0 | 0.6 | 120 | 4 | Cross | 1.55 | 2.3 | 0.47 | 311 | 3,550 |
| Comparative Example 5 | Figure 5 | 0.10 | 1.0 | 10.0 | 0.6 | 120 | 4 | Cross | 1.55 | 2.3 | 0.47 | 311 | 3.745 |
| Example 13 | Figure 5 | 0.10 | 1.0 | 10.0 | 0.6 | 120 | 4 | Cross | 1.55 | 2.3 | 0.47 | 311 | 3,582 |

(continued)

| | Fiber (max) | | | | | | | | | | | |
| | Shape & Size of Spinneret | | | | Extrusion Rate (cc/min) | Equivalent Circle Diameter (μm) | Protrusion Numbers at Fiber Cross section (pieces) | Fiber Cross-sectional Shape | Surface Area Increase Rate | Modification Degree (Outer/Inner ) | Occupancy Rate against a Circumscribed Circle | Specific Surface Area (m²/g) | Numbers of Fibers (strands) |
| | Shape of Spinneret (Figure No.) | W (mm) | L (mm) | L/W | | | | | | | | | |
| Example 14 | Figure 5 | 0.10 | 1.0 | 10.0 | 0.6 | 120 | 4 | Cross | 1.55 | 2.3 | 0.47 | 311 | 3,089 |
| Example 15 | Figure 5 | 0.10 | 1.0 | 10.0 | 0.6 | 120 | 4 | Cross | 1.55 | 2.3 | 0.47 | 311 | 2,222 |
| Example 16 | Figure 5 | 0.10 | 1.0 | 10.0 | 0.6 | 120 | 4 | Cross | 1.55 | 2.3 | 0.47 | 311 | 1,657 |
| Example 17 | Figure 5 | 0.10 | 1.0 | 10.0 | 0.6 | 120 | 4 | Cross | 1.55 | 2.3 | 0.47 | 311 | 604 |
| Example 18 | Figure 2 | 0.10 | 1.0 | 10.0 | 0.6 | 120 | 3 | Y-shape | 1.55 | 3.0 | 0.32 | 308 | 2,600 |
| Example 19 | Figure 7 | 0.10 | 1.0 | 10.0 | 0.6 | 120 | 5 | Star | 1.55 | 1.75 | 0.63 | (≥250) | 2,600 |
| Example 20 | Figure 5 | 0.10 | 1.0 | 10.0 | 0.6 | 120 | 4 | Cross | 1.55 | 2.3 | 0.47 | 311 | 2,600 |
| Example 21 | Figure 5 | 0.10 | 1.0 | 10.0 | 0.6 | 120 | 4 | Cross | 1.55 | 2.3 | 0.47 | 311 | 2,600 |
| Comparative Example 6 | Circle | - | - | - | - | 120 | 0 | Circle | 1.00 | 1.0 | 1.0 | 0.1 | 3,900 |

[Table 2]

| | Fiber (min) | | | | | | | | | | | | |
| | Shape & Size of Spinneret | | | | Extrusion Rate (cc/min) | Equivalent Circle Diameter (μm) | Protrusion Numbers at Fiber Cross section (pieces) | Fiber Cross-sectional Shape | Surface Area Increase Rate | Modification Deg ree (Outer/Inner ) | Occupancy Rate against a Circumscribed Circle | Specific Surface Area (m²/g) | Numbers of Fibers (strands) |
| | Shape of Spinneret (Figure No.) | W (mm) | L (mm) | L/W | | | | | | | | | |
| Example 1 | Circle | - | - | - | 0.6 | 120 | 0 | Circle | 1.00 | 1.0 | 1.0 | 305 | 1,300 |
| Example 2 | Figure 4 | 0.10 | 0.6 | 6.0 | 0.6 | 120 | 0 | Oval | 1.10 | 1.73 | 0.66 | (≥250) | 1,300 |
| Example 3 | Figure 4 | 0.10 | 1.0 | 10.0 | 0.6 | 120 | 0 | Oval | 1.20 | 2.6 | 0.42 | 299 | 1,300 |
| Example 4 | Figure 4 | 0.10 | 1.2 | 12.0 | 0.6 | 120 | 0 | Oval | 1.30 | 3.45 | 0.31 | (≥250) | 1,300 |
| Example 5 | Figure 4 | 0.10 | 1.4 | 14.0 | 0.6 | 120 | 0 | Oval | 1.40 | 4.39 | 0.2 | (≥250) | 1,300 |
| Comparative Example 1 | - | - | - | - | - | - | - | - | - | - | - | - | 0 |
| Comparative Example 2 | - | - | - | - | - | - | - | - | - | - | - | - | 0 |
| Comparative Example 3 | - | - | - | - | - | - | - | - | - | - | - | - | 0 |
| Comparative Example 4 | Figure 4 | 0.10 | 1.0 | 10.0 | 0.6 | 120 | 0 | Oval | 1.20 | 2.6 | 0.42 | 299 | 650 |
| Example 6 | Figure 4 | 0.10 | 1.0 | 10.0 | 0.6 | 120 | 0 | Oval | 1.20 | 2.6 | 0.42 | 299 | 1,300 |
| Example 7 | Figure 4 | 0.10 | 1.0 | 10.0 | 0.6 | 120 | 0 | Oval | 1.20 | 2.6 | 0.42 | 299 | 1,950 |
| Example 8 | Figure 4 | 0.10 | 1.0 | 10.0 | 0.6 | 120 | 0 | Oval | 1.20 | 2.6 | 0.42 | 299 | 1,110 |
| Example 9 | Figure 4 | 0.10 | 1.0 | 10.0 | 0.6 | 120 | 0 | Oval | 1.20 | 2.6 | 0.42 | 299 | 975 |
| Example 10 | Figure 4 | 0.10 | 1.0 | 10.0 | 0.6 | 120 | 0 | Oval | 1.20 | 2.6 | 0.42 | 299 | 650 |
| Example 11 | Figure 4 | 0.10 | 1.0 | 10.0 | 0.6 | 120 | 0 | Oval | 1.20 | 2.6 | 0.42 | 299 | 430 |
| Example 12 | Figure 4 | 0.10 | 1.0 | 10.0 | 0.6 | 120 | 0 | Oval | 1.20 | 2.6 | 0.42 | 299 | 350 |
| Comparative Example 5 | Figure 4 | 0.10 | 1.0 | 10.0 | 0.6 | 120 | 0 | Oval | 1.20 | 2.6 | 0.42 | 299 | 155 |
| Example 13 | Figure 4 | 0.10 | 1.0 | 10.0 | 0.31 | 55 | 0 | Oval | 1.20 | 2.6 | 0.42 | (≥250) | 1,791 |

(continued)

| | Fiber (min) | | | | | | | | | | | | |
| | Shape & Size of Spinneret | | | | Extrusion Rate (cc/min) | Equivalent Circle Diameter (µm) | Protrusion Numbers at Fiber Cross section (pieces) | Fiber Cross-sectional Shape | Surface Area Increase Rate | Modification Degree (Outer/Inner) | Occupancy Rate against a Circumscribed Circle | Specific Surface Area (m²/g) | Numbers of Fibers (strands) |
| | Shape of Spinneret (Figure No.) | W (mm) | L (mm) | L/W | | | | | | | | | |
| Example 14 | Figure 4 | 0.10 | 1.0 | 10.0 | 0.42 | 90 | 0 | Oval | 1.20 | 2.6 | 0.42 | (≥250) | 1,545 |
| Example 15 | Figure 4 | 0.10 | 1.0 | 10.0 | 0.89 | 150 | 0 | Oval | 1.20 | 2.6 | 0.42 | (≥250) | 1,111 |
| Example 16 | Figure 4 | 0.10 | 1.0 | 10.0 | 1.21 | 200 | 0 | Oval | 1.20 | 2.6 | 0.42 | (≥250) | 828 |
| Example 17 | Figure 4 | 0.10 | 1.0 | 10.0 | 4.23 | 400 | 0 | Oval | 1.20 | 2.6 | 0.42 | (≥250) | 302 |
| Example 18 | Figure 4 | 0.10 | 1.0 | 10.0 | 0.6 | 120 | 0 | Oval | 1.20 | 2.6 | 0.42 | 299 | 1,300 |
| Example 19 | Figure 4 | 0.10 | 1.0 | 10.0 | 0.6 | 120 | 0 | Oval | 1.20 | 2.6 | 0.42 | 299 | 1,300 |
| Example 20 | Figure 2 | 0.10 | 1.0 | 10.0 | 0.6 | 120 | 3 | Y-shape | 1.20 | 1.9 | 0.79 | 308 | 1,300 |
| Example 21 | Figure 6 | 0.10 | 1.0 | 10.0 | 0.6 | 120 | 5 | Star | 1.20 | 1.3 | 0.92 | (≥250) | 1,300 |
| Comparative Example 6 | - | - | - | - | - | - | - | - | - | - | - | - | 0 |

[Table 3]

| | Mixed Fiber Bundle | | | | | |
| --- | --- | --- | --- | --- | --- | --- |
| | Fiber Diameter Ratio Y | Sfo Ratio Z | Mixing Ratio of Fiber$_{(max)}$ vs Fiber$_{(min)}$ | Ratio of Fiber $_{(max)}$ (%) | Ratio of Fiber (min) (%) | Adsorption Performance per Volume of Fiber (mg/cm$^3$) |
| Example 1 | 1.00 | 0.47 | 2 :1 | 66.7 | 33.3 | No data |
| Example 2 | 1.00 | 0.71 | 2 :1 | 66.7 | 33.3 | No data |
| Example 3 | 1.00 | 1.12 | 2 :1 | 66.7 | 33.3 | 0.038 |
| Example 4 | 1.00 | 1.52 | 2 :1 | 66.7 | 33.3 | No data |
| Example 5 | 1.00 | 2.35 | 2 :1 | 66.7 | 33.3 | No data |
| Comparative Example 1 | - | - | 1 :0 | 100.0 | 0.0 | No data |
| Comparative Example 2 | - | - | 1 :0 | 100.0 | 0.0 | No data |
| Comparative Example 3 | - | - | 1 :0 | 100.0 | 0.0 | No data |
| Comparative Example 4 | 1.00 | 1.12 | 1 :5 | 83.3 | 16.7 | No data |
| Example 6 | 1.00 | 1.12 | 1 2 | 66.7 | 33.3 | No data |
| Example 7 | 1.00 | 1.12 | 1 :1 | 50.0 | 50.0 | No data |
| Example 8 | 1.00 | 1.12 | 2.5 :1 | 71.5 | 28.5 | No data |
| Example 9 | 1.00 | 1.12 | 3 :1 | 75.0 | 25.0 | No data |
| Example 10 | 1.00 | 1.12 | 5 :1 | 83.3 | 16.7 | No data |
| Example 11 | 1.00 | 1.12 | 8 :1 | 89.0 | 11.0 | No data |
| Example 12 | 1.00 | 1.12 | 10 :1 | 91.0 | 9.0 | No data |
| Comparative Example 5 | 1.00 | 1.12 | 24 :1 | 96.0 | 4.0 | No data |
| Example 13 | 0.46 | 1.12 | 2 :1 | 66.7 | 33.3 | No data |
| Example 14 | 0.75 | 1.12 | 2 :1 | 66.7 | 33.3 | No data |
| Example 15 | 1.25 | 1.12 | 2 :1 | 66.7 | 33.3 | No data |
| Example 16 | 1.67 | 1.12 | 2 :1 | 66.7 | 33.3 | No data |
| Example 17 | 3.33 | 1.12 | 2 :1 | 66.7 | 33.3 | No data |
| Example 18 | 1.00 | 0.76 | 2 :1 | 66.7 | 33.3 | No data |
| Example 19 | 1.00 | 1.50 | 2 :1 | 66.7 | 33.3 | No data |
| Example 20 | 1.00 | 0.59 | 2 :1 | 66.7 | 33.3 | No data |
| Example 21 | 1.00 | 0.51 | 2 :1 | 66.7 | 33.3 | No data |
| Comparative Example 6 | - | - | 1 :0 | 100.0 | 0.0 | 0.001 |

[Table 4]

| | Purification Column | | |
|---|---|---|---|
| | Filling Ratio (%) | Co (ml/min) | Ko (cm/min) |
| Example 1 | 57 | 1.03 | 0.0017 |
| Example 2 | 57 | 1.33 | 0.0019 |
| Example 3 | 57 | 1.53 | 0.0021 |
| Example 4 | 57 | 1.56 | 0.0021 |
| Example 5 | 57 | 1.65 | 0.0021 |
| Comparative Example 1 | 57 | 1.13 | 0.0014 |
| Comparative Example 2 | 57 | 0.87 | 0.0014 |
| Comparative Example 3 | 57 | 0.79 | 0.0015 |
| Comparative Example 4 | 57 | 1.15 | 0.0015 |
| Example 6 | 57 | 1.36 | 0.0019 |
| Example 7 | 57 | 1.37 | 0.0019 |
| Example 8 | 57 | 1.57 | 0.0021 |
| Example 9 | 57 | 1.52 | 0.0020 |
| Example 10 | 57 | 1.55 | 0.0020 |
| Example 11 | 57 | 1.45 | 0.0019 |
| Example 12 | 57 | 1.40 | 0.0018 |
| Comparative Example 5 | 57 | 1.13 | 0.0014 |
| Example 13 | 57 | 1.68 | 0.0020 |
| Example 14 | 57 | 1.59 | 0.0020 |
| Example 15 | 57 | 1.30 | 0.0019 |
| Example 16 | 57 | 1.05 | 0.0019 |
| Example 17 | 57 | 0.51 | 0.0018 |
| Example 18 | 57 | 1.49 | 0.0020 |
| Example 19 | 57 | 1.55 | 0.0021 |
| Example 20 | 57 | 1.37 | 0.0019 |
| Example 21 | 57 | 1.29 | 0.0018 |
| Comparative Example 6 | 57 | 0.02 | 0.0000 |

The results of Examples 1 to 5 and Comparative Examples 1 to 3 indicate that a higher value of the adsorption performance Co is shown in the columns each incorporating a blended fibrous material that is composed of fibers with a cross-shaped cross section as fibers$_{(max)}$ and fibers with either an oval or round cross section as fibers$_{(min)}$, as compared with the columns each incorporating a fibrous material composed of a single type of fibers, such as fibers with a cross-shaped cross section, fibers with an oval cross section, or fibers with a round cross section. Moreover, the fibers$_{(min)}$ having a higher modification degree and a lower occupancy rate against a circumscribed showed a tendency to have a higher value of Ko, an index of adsorption performance per unit surface area. Thus, it is considered that, if fibers$_{(min)}$ have either a round or oval cross section, a rise in surface area increase rate leads to an increase of the surface area in the resulting fibrous material and, moreover, a higher modification degree and a lower occupancy rate against a circumscribed circle result in a higher preventative effect on adhesion of fibers.

The results of Example 3 and Examples 6 to 12 and Comparative Examples 4 and 5 indicated a tendency of the fibrous materials containing different types of fibers at a mixing ratio (fiber$_{(max)}$ : fiber$_{(min)}$) from 1:2 to 10:1 to have values of Co and Ko higher than those of Comparative Example 1, in which fibers with a cross-shaped cross section were used alone, or those of Comparative Example 2, in which fibers with an oval cross section were used alone. On the other hand, the fibrous material of Comparative Example 4, which had been obtained by using the fibers$_{(max)}$ at a ratio as small as 17%, had a value of Co lower than that of Comparative Example 1, in which fibers with a cross-shaped cross section were used alone, and a value of Ko almost equal to those of Comparative Examples 1 to 3. This is likely due to an insufficient effect of the fiber$_{(max)}$ to increase the surface area as well as due to adhesion of the fibers$_{(min)}$ to each other and the resulting decreased availability of the surface area of the fibers. In Comparative Example 5, the ratio of the fiber$_{(min)}$ to the total fibers was as small as 4% and the preventative effect on adhesion of fibers was insufficient, which caused to lose the effect of fiber blending, as indicated by a value of Ko as low as that of Comparative Example 1, in which fibers with an oval cross section were used alone.

The results of Example 3 and Examples 13 to 17 indicate that a too large equivalent circle diameter of a fiber$_{(min)}$ as compared to that of a fiber$_{(max)}$ results in an increased surface area loss and a decreasing tendency in adsorption performance.

The results of Examples 3, 18 and 19 indicate that an increased occupancy rate against the circumscribed circle of a fiber$_{(max)}$ leads to an improvement in adsorption performance. This is likely due to an enhanced preventative effect on adhesion of fibers, which is caused by the increased occupancy rate against the circumscribed circle of the fiber$_{(max)}$ and the resulting decrease in fibers$_{(max)}$ overlapping each other in such a manner that a protrusion of a fiber engages a trough of another neighboring fiber.

The results of Examples 3, 20 and 21, in which the cross-sectional shape of fibers$_{(min)}$ is changed, indicate that higher performance is achieved by fibers$_{(min)}$ with a Y-shaped cross-sectional shape than by those with a star-shaped cross-sectional shape, and further indicate that higher values of Co and Ko are obtained by fibers$_{(min)}$ with an oval cross-sectional shape than by those with a Y-shaped cross-sectional shape. This suggests that a fiber$_{(min)}$ having a cross-sectional shape with a higher modification degree and a lower occupancy rate against a circumscribed circle is more advantageous. This is likely due to an enhanced preventative effect on adhesion of fibers, which is caused by the decreased occupancy rate against the circumscribed circle of the fiber$_{(min)}$.

Moreover, the results of Example 3 and Comparative Example 6 indicate that the porous fibrous material used in Example 3 has a higher level of adsorption performance per volume of fiber and a sufficient protein adsorption activity, as compared with the filament having a small specific surface area as in Comparative Example 6.

Example 22

<Production of a column>

The same filament as in Example 3 was used to produce a fibrous material composed of 50,000 fibers$_{(max)}$ and 25,000 fibers$_{(min)}$ and the resulting fibrous material was incorporated in a straight form into a cylindrical casing made of polycarbonate with an inner diameter of 46 mm and an axial length of 140 mm to give a filling ratio of fibers at 57%. Then, the outlet and inlet openings for a fluid to be treated at the ends on both sides of the casing were covered with mesh filters with an opening equivalent diameter of 84 $\mu$m and an aperture ratio of 36%, which mesh filters were cut into a shape having the same diameter as the inner diameter of the casing. Finally, caps with inlet and outlet ports for a fluid to be treated, which are called headers, were attached on the ends of the casing. The equivalent circle diameter, the surface area increase rate, and the average pore radius were measured in the obtained filament by the above-described methods. Moreover, the adsorption performance, the pressure loss, and the volume of remaining blood were measured in the resulting column by the methods as described below. The results are presented in Table 5.

[Table 5]

| | | Example 22 | | Comparative Example 7 | Comparative Example 8 | Example 23 | | Reference Example 1 | | Reference Example 2 | Reference Example 3 | Reference Example 4 | Reference Example 5 |
|---|---|---|---|---|---|---|---|---|---|---|---|---|---|
| | | Fiber$_{(max)}$ | Fiber i-iD-er$_{(min)}$ | | | Fiber$_{(max)}$ | Fiber$_{(min)}$ | Fiber$_{(max)}$ | Fiber$_{(min)}$ | | | | |
| Solid Fiber | Equivalent Circle Diameter [$\mu$m] | 120 | 120 | 120 | 120 | 120 | 120 | 290 | 290 | 113 | 113 | 113 | 113 |
| | Fiber Cross-sectional Shape [-] | Cross | Oval | Cross | Oval | Cross | Oval | Cross | Oval | Circle | Circle | Circle | Circle |
| | Surface Area Increase Rate [-] | 1.55 | 1.20 | 1.55 | 1.20 | 1.55 | 1.20 | 1.55 | 1.20 | 1.00 | 1.00 | 1.00 | 1.00 |
| | Numbers of Fibers [strands] | 50,000 | 25,000 | 75,000 | 75,000 | 27,000 | 13,500 | 4,700 | 2,350 | 60,000 | 82,000 | 40,000 | 4,200,000 |
| | Membrane Surface [m$^2$] | 5.7 | | 6.1 | 4.7 | 3.1 | | 1.3 | | 3.0 | 4.1 | 2.0 | 3.1 |
| | Average Pore Radius [nm] | 9 | 8 | 9 | 8 | 9 | 8 | 9 | 8 | 12 | 12 | 12 | 12 |

(continued)

| | | Example 22 | | Comparative Example 7 | Comparative Example 8 | Example 23 | | Reference Example 1 | | Reference Example 2 | Reference Example 3 | Reference Example 4 | Reference Example 5 |
|---|---|---|---|---|---|---|---|---|---|---|---|---|---|
| | | Fiber$_{(max)}$ | Fiber i-iD-er$_{(min)}$ | | | Fiber$_{(max)}$ | Fiber$_{(min)}$ | Fiber$_{(max)}$ | Fiber$_{(min)}$ | | | | |
| | Inner Diameter of Case [mm] | 46 | | 46 | 46 | 46 | | 46 | | 46 | 46 | 46 | 46 |
| | Filling Ratio [%] | 51 | | 51 | 51 | 28 | | 28 | | 36 | 49 | 24 | 36 |
| | Volume of Blood (Case Part) [ml] | 114 | | 114 | 114 | 169 | | 167 | | 149 | 118 | 178 | 149 |
| | Equivalent Diameter of Flow Cross section [μm] | 80 | | 74 | 96 | 219 | | 512 | | 198 | 115 | 352 | - |
| | Pressure Loss [kPa] | 34 | | 40 | 37 | 13 | | 1.4 | | 21.6 | 27.0 | 6.3 | 6.0 |
| | Effective Fiber Length [mm] | 140 | | 140 | 140 | 140 | | 140 | | 140 | 140 | 140 | 20 |
| Column | Measured Value of β$_2$-MG Clearance Co | 94 | | 75 | 59 | 56 | | 15 | | 51 | 71 | 23 | 36 |
| | Ko [cm/min] | 0.0022 | | 0.0015 | 0.0015 | 0.0021 | | 0.0012 | | 0.0020 | 0.0021 | 0.0012 | 0.0013 |
| | β$_2$-MG clearance (3m² Converted Value) | 57 | | 41 | 40 | 55 | | 33 | | 51 | 55 | 33 | 35 |
| | Volume of Remaining Blood [ml] | 0.5 | | 1.0 | 1.0 | 3.6 | | 3.9 | | 3.5 | 0.9 | 6.5 | 12.0 |

<Measurement of the adsorption performance of a column>

The clearance of $\beta_2$-MG was measured to evaluate the adsorption performance. Bovine blood supplemented with disodium ethylenediaminetetraacetate was adjusted to have a hematocrit of 30 ± 3% and a total protein concentration of 6.5 ± 0.5 g/dL. The bovine blood stored for up to five days after blood collection was used.

Then, $\beta_2$-MG was added to a concentration of 1 mg/L and the resulting mixture was stirred. The bovine blood was divided two portions: one having a volume of 2 L for circulation and the other having a volume of 1.5 L for clearance measurement.

A circuit was prepared as shown in Fig. 7. In the circuit, a port for introducing a fluid to be treated was designated as Bi and a port for discharging a fluid passing through a purification column was designated as Bo.

The Bi was placed into a beaker for a circulating fluid, which contained 2 L (at 37°C) of the above adjusted bovine blood, and pumping at a flow rate of 200 mL/min was started. Immediately after discarding a volume of a fluid that was discharged from the Bo for the first 90 seconds, the Bo was placed into the beaker for a circulating fluid to establish the circulation. The circulation of the fluid was continued for one hour and then the pump was stopped.

Next, the Bi was placed into the above adjusted bovine blood for clearance measurement and the Bo was placed into a beaker for waste.

Two minutes after starting pumping at a flow rate of 200 mL/min, an aliquot of 10 mL was withdrawn from the bovine blood (at 37°C) for clearance measurement and designated as Bi liquid. Four minutes and thirty seconds after starting pumping, 10 mL of a sample discharged from the Bo was collected and designated as Bo liquid. These samples were stored in a freezer at -20°C or lower.

The clearance Co was calculated from the concentration of $\beta_2$-MG in each of the liquids. Since measurement values could vary depending on each bovine blood lot, the same lot of bovine blood was used in all of Examples and Comparative Examples.

The result is presented in Table 5.

<Measurement of pressure loss using bovine blood>

In the above-described measurement of the adsorption performance of a column, the pressure was measured at the Bi and the Bo at five minutes after starting the clearance measurement and the difference in pressure between the Bi and the Bo was considered to be the pressure loss. The result is presented in Table 5.

<Test of blood reinfusion property>

An adsorption column was washed with 700 mL of saline that flowed from the top to the bottom of the column at a flow rate of 200 mL/min. During the washing, air bubbles were not tried to be removed by, for example, applying vibration to the adsorption column.

Then, bovine blood was introduced into the adsorption column from the bottom at a flow rate of 200 mL/min. Bovine blood supplemented with heparin and adjusted to have a hematocrit of 30% and a total protein concentration of 6.5 g/dL was used. Once the bovine blood was confirmed to appear in the header on the top of the adsorption column, the adsorption column was inverted upside down to cause the blood to flow from the top to the bottom of the column. The condition was maintained to allow the blood to circulate for one hour.

For blood reinfusion, two to three column volumes of saline was allowed to flow from the top to the bottom of the adsorption column in the one-path format at a flow rate of 100 mL/min. Aliquots of the blood reinfusion fluid discharged from the bottom of the column were sampled over time and the last 100 mL of the blood reinfusion fluid (if the column volume is 100 mL, it corresponds to a fluid collected from the column while the volume of saline flowing through the column is increased from 200 to 300 mL) was diluted twice with pure water to induce hemolysis. Absorbance at a wavelength of 414 nm was measured on a ultra-violet and visible spectrophotometer (UV-160; manufactured by Shimadzu Corporation) to calculate the amount of hemoglobin contained in the fluid, which was then converted to the volume of remaining blood in the column. A standard curve was generated using a bovine blood standard adjusted to have a hematocrit of 30% and a total protein concentration of 6.5 g/dL. The result is presented in Table 5.

Example 23

A column was produced and then evaluated by the same methods as in Example 22 by using the same fibers and the same casing as in Example 22, except that the numbers of fibers$_{(max)}$ and fibers$_{(min)}$ were 27,000 and 13,500, respectively. The result is presented in Table 5.

Comparative Example 7

A column was produced and then evaluated by the same methods as in Example 22 by using the same fiber as in Comparative Example 1, except that the number of fibers was 75,000. The result is presented in Table 5.

Comparative Example 8

A column was produced and then evaluated by the same methods as in Example 22 by using the same fiber as in Comparative Example 2, except that the number of fibers was 75,000. The result is presented in Table 5.

Reference Example 1

A column was produced and then evaluated by the same methods as in Example 22 by using the same fibers and the same casing as in Example 22, except that the numbers of fibers$_{(max)}$ and fibers($_{min}$) were 13,500 and 6,750, respectively. The result is presented in Table 5.

Reference Example 2

A fibrous material was produced under the same conditions as in Example 1 to obtain a fiber having a round cross section with an equivalent diameter of 113 $\mu$m, except that only a spinneret having an orifice of a round shape was installed and the extrusion rate was 0.5 cc/min and the temperature of a coagulating bath was 48°C. Into a casing having an inner diameter of 46 mm, 60,000 lines of the solid fiber having an appropriate length of 140 mm was installed to prepare a column. The column was evaluated similarly to Example 22. As shown in Table 5, a high clearance of $\beta_2$-MG and a satisfactory blood remaining property (a small volume of remaining blood) were obtained. Moreover, the result of a pulse test performed by the procedure as described below indicated the peak top located at 0.77, which suggested a satisfactory distribution of flow.

<Pulse test>

A pulse test was performed to quantify the uniformity in distribution of flow of a fluid that is to be treated in a column. The column was connected to a circuit and the circuit and the adsorption column were washed by allowing 1 L of ultrapure water to flow at a flow rate of 200 mL/min. An injection of a 1-mL aliquot of a 110 times dilution of India ink into a port of the circuit was completed within two seconds by using a syringe.
The time of starting India ink injection was defined as the time point of 0 second and sampling of 3-mL aliquots at an interval of one second was started three seconds later (100 seconds and 98 samples in total). A spectrophotometer (U-2000; manufactured by Hitachi High-Technologies Corporation) was used to measure the concentration of India ink at a wavelength of 600 nm. After generating a standard curve, the samples were measured.
The obtained data was used to generate a scatter plot by plotting a space-time ($\varphi$) on the X axis and a dimensionless concentration (E) on the Y axis and the space-time ($\varphi$) of a sample that exhibited the highest dimensionless concentration (E) was considered as the peak top. As used herein, $\varphi$ and E are given by the following formulae formula (15) and (16), respectively. The results are presented in Table 5.

$$\varphi = t/TI = t / (VI/v) \tag{15}$$

In the above-described formula (15), TI represents the theoretical column transition time, t represents the sampling time (the time of starting India ink injection is defined as the time point of 0 sec), VI represents the theoretical column volume, and v represents the flow rate (200 ml/sec).

$$E = C/C_0 \tag{16}$$

In the above-described formula (16), C represents the concentration of India ink in each sampled solution, and $C_0$ represents the initial concentration of India ink.

Reference Example 3

A column was produced by using the same fiber and the same casing as in Reference Example 2 were used, except that the number of installed fibers was 82,000. The column was likewise evaluated similarly to Reference Example 2.

As shown in Table 5, a high clearance of $\beta_2$-MG and a satisfactory blood remaining property (a small volume of remaining blood) were obtained. A large pressure loss as compared to that in Reference Example 2 likely leads to a value of $\beta_2$-MG clearance higher than that of Example 1. Moreover, the result of the pulse test indicated the peak top located at 0.80, which suggested a satisfactory distribution of flow.

Reference Example 4

A column was produced by using the same fiber and the same casing as in Reference Example 2, except that the number of installed solid fibers was 40,000. The column was likewise evaluated similarly to Reference Example 2. The column was evaluated similarly to Example 22. As shown in Table 5, a lower clearance of $\beta_2$-MG and a poorer blood remaining property as compared to those of Reference Example 2 were obtained. A small pressure loss likely leads to the lower clearance of $\beta_2$-MG and the large volume of remaining blood. Moreover, the result of the pulse test indicated the peak top located at 0.28, which also suggested a less even distribution of flow.

Reference Example 5

The same fiber as in Reference Example 2 was cut to a length of about 20 mm and the thus prepared about 4,200,000 lines of the fiber were randomly installed into a casing having an inner diameter of 46 mm to prepare an adsorption column. The column was evaluated similarly to Example 22. As shown in Table 5, a low clearance of $\beta_2$-MG and a large volume of remaining blood were obtained.

As shown above, the results of Example 22 and Comparative Examples 7 and 8 indicate that a high clearance of $\beta_2$-MG is shown in the column incorporating a fibrous material that is composed of fibers with a cross-shaped cross section as fibers$_{(max)}$ and fibers with an oval cross section as fibers(min), as compared with the columns each incorporating a fibrous material composed of a single type of fibers, such as fibers with a cross-shaped cross section or fibers with an oval cross section.

The results of Examples 22 and 23 and Reference Example 1 indicate that sufficient adsorption performance can be provided when the equivalent diameter of flow cross-section is not more than 250 $\mu$m. Moreover, satisfactory volumes of remaining blood are also indicated.

Moreover, the results of Reference Examples 2 to 4 indicate that sufficient adsorption performance can be provided when the pressure loss is preferably not less than 0.5 kPa. Moreover, satisfactory pulse test results and satisfactory volumes of remaining blood are also indicated.

Furthermore, the results of Reference Examples 2 and 5 indicate that installation of fibers as a straight bundle, rather than random installation of fibers, can enhance the adsorption performance and reduce the volume of remaining blood.

No macrovoids were confirmed in fibers used in Examples, Comparative Examples and Reference Examples of the present application.

INDUSTRIAL APPLICABILITY

A fibrous material according to the present invention efficiently adsorbs and removes substances to be adsorbed in a fluid that is to be treated and therefore can be used in a purification column. The purification column has a variety of specific uses and can be used for various applications such as water treatment, purification, and medical treatment.

DESCRIPTION OF SYMBOLS

1     Circumscribed circle
2     Inscribed circle
3     Diameter of a circumscribed circle *Do*
4     Diameter of an inscribed circle *Di*
10    Length of a slit
11    Width of a slit
R     Circular arc
W    Width of a slit
L     Length of a slit

13    Purification column
14    Pump
15    Water bath at 37°C
16    Beaker for waste
17    Plasma for circulation
18    Plasma for clearance measurement


**Claims**

1.    A fibrous material comprising polymeric fiber blends having plural types of solid fibers having a common cross-sectional shape, wherein the raw material of the polymeric fiber is selected from the group consisting of polymethylmethacrylate, polyester, polyacrylonitrile, polysulfone, polyethersulfone, polyarylethersulfone, polypropylene, polystyrene, polycarbonate, cellulose, cellulose triacetate, and ethylene-vinyl alcohol copolymer,

wherein fibers are considered to have a common cross-sectional shape when the cross-section of two fibers are compared and the comparison identifies that none of the following points (i) to (vi) is met,

(i) one of the fibers has a protrusion at the periphery of the crosssection and the other fiber does not have a protrusion at the periphery of the cross section,
(ii) the number of protrusions is different between the fibers,
(iii) when the fibers have the same number of protrusions and, have a difference of not less than 30% in the surface area increase rate as determined by the method of the description,
(iv) a fiber having a round or oval cross section, among fibers having no protrusion, is compared with another fiber similarly having a round or oval cross section and the comparison identifies those fibers as having a difference of not less than 30% in the surface area increase rate or as having a difference of not less than 50% in modification degree as determined by the method of the description,
(v) a fiber having a round or oval cross section and a fiber having an angular cross section,
(vi) when each of the fibers has an angular cross section, the fibers have cross sections with different numbers of angles,
(vii) when the fibers having angular cross sections with the same number of angles and, have a difference of not less than 30% in the surface area increase rate,

wherein the average pore radius of the solid fibers is within the range of 1 to 100 nm as determined by the method of the description,
wherein the composition ratio of each of at least two out of the plural types of fibers to the total of the fiber blends is not less than 5.0%, and when, among those plural types of fibers having a composition ratio of not less than 5.0%, fibers having the highest and the lowest surface area increase rates, which are given by the formula (1) below, are respectively designated as the fiber$_{(max)}$ and the fiber$_{(min)}$, the surface area increase rate of the fiber$_{(min)}$ is reduced by 3.0% or more as compared to that of the fiber$_{(max)}$, wherein the occupancy rate $Sfo$ against the circumscribed circle of the fiber$_{(min)}$, which is given by the formula (3) below, is not more than 0.90, wherein in the formula (3) $Sf$ represents the cross-sectional area of a fiber cross section and So represents the area of the circumscribed circle of the fiber cross section, wherein the modification degree $Do/Di$ of the fiber$_{(min)}$ is not less than 1.10 and not more than 6.00, wherein $Do$ represents the diameter of the circumscribed circle of a fiber cross section and $Di$ represents the diameter of the inscribed circle of the fiber cross section, and the composition ratios of the fiber$_{(max)}$ and the fiber$_{(min)}$ to the total of the fiber blends, which is given by the formula (2), are not less than 30.0% and not less than 8.0%, respectively, and the fiber$_{(max)}$ has (a) a surface area increase rate of not less than 1.20 and is (b) a porous fiber with a micropore specific surface area, which is obtained through the measurement of the degree of freezing point depression of water in micropores induced by capillary condensation by differential scanning calorimetry using a differential scanning calorimeter, of not less than 10 m$^2$/g, wherein the fiber diameter ratio Y, which is obtained by dividing the equivalent circle diameter of the fiber$_{(min)}$ by the equivalent circle diameter of the fiber$_{(max)}$, is not less than 0.2 and not more than 10.0, wherein the equivalent circle diameter of the fiber$_{(min)}$ and the equivalent circle diameter of the fiber$_{(max)}$ are given by the formula (4), wherein S in the formula (4) represents the cross-sectional area of a fiber; wherein

$$\text{Surface area increase rate} = (\text{the circumference of a fiber cross section}) / (\text{the circumference of a circle having the same cross-sectional area as the fiber cross section}) \quad (1)$$

$$\text{Composition ratio in the total of fiber blend materials (\%)} = \text{the number of fibers having a common cross-sectional shape} / \text{total number of fibers x 100} \quad (2)$$

$$\text{Occupancy rate } Sfo \text{ against a circumscribed circle} = Sf/So \quad (3)$$

$$\text{Equivalent circle diameter of a fiber crosssection} = 2 \times (S/\pi)^{1/2} \quad (4).$$

2. The fibrous material according to claim 1 , wherein the fiber$_{(min)}$ is a porous fiber with a micropore specific surface area, which is obtained through the measurement of the degree of freezing point depression of water in micropores induced by capillary condensation by differential scanning calorimetry using a differential scanning calorimeter, of not less than 5 m$^2$/g.

3. The fibrous material according to claim 2, wherein the fiber(min) is a porous fiber with a micropore specific surface area of not less than 10 m$^2$/g.

4. The fibrous material according to any one of claims 1 to 3, wherein the surface area increase rate of the fiber$_{(min)}$ is not less than 1.10.

5. The fibrous material according to any one of claims 1 to 4, wherein the fiber cross-sectional shape of the fiber$_{(min)}$ is round or oval.

6. The fibrous material according to any one of claims 1 to 5, wherein the equivalent circle diameter, which is given by the formula (4), of the fiber$_{(max)}$ is not less than 10 $\mu$m and not more than 1,000 $\mu$m.

7. The fibrous material according to any one of claims 1 to 6, wherein the $Sfo$ ratio Z obtained by dividing the occupancy rate $Sfo$ against the circumscribed circle of the fiber$_{(max)}$ by the occupancy rate $Sfo$ against the circumscribed circle of the fiber$_{(min)}$ is not less than 0.20.

8. The fibrous material according to any one of claims 1 to 7, which is composed of straight fibers.

9. The fibrous material according to any one of claims 1 to 8, wherein the fiber blend is composed of two types of fibers having a common cross-sectional shape.

10. The fibrous material according to any one of claims 1 to 9, wherein the fiber$_{(max)}$ and the fiber$_{(min)}$ are produced from the same raw material.

11. The fibrous material according to claim 9 or 10, wherein the raw material is an ester group-containing polymer.

12. A purification column comprising a plastic casing and the fibrous material according to any one of claims 1 to 11, in which the fibrous material is arranged in the plastic casing straight in the axial direction extending between openings on both sides of the casing, and an inlet port and an outlet port for a fluid that is to be treated are provided at either end of the plastic casing; and

   wherein the equivalent diameter of flow cross-section, which is given by the formula (6) below, is not less than 10 $\mu$m and not more than 250 $\mu$m,

$$D_p = 4 \times ((D_{case}/2)^2 - (D_{fiber\text{-}max}/2)^2 \times N_{max} - (D_{fiber\text{-}min}/2)^2 \times N_{min}) / (D_{case} + D_{fiber\text{-}max} \times$$

$$SA_{max} \times N_{max} + D_{fiber\text{-}min} \times SA_{min} \times N_{min}) \hspace{3cm} (6),$$

wherein in the formula (6), $D_{case}$ represents the inner diameter of a column casing, and Dfiber-max represents the equivalent circle diameter of a fiber$_{(max)}$, $D_{fiber\text{-}min}$ represents the equivalent circle diameter of a fiber$_{(min)}$, $N_{max}$ represents the number of fibers$_{(max)}$, $N_{min}$ represents the number of fibers(min), SA$_{max}$ represents the surface area increase rate of a fiber$_{(max)}$ and SA$_{min}$ represents the surface area increase rate of a fiber$_{(min)}$; and wherein the pressure loss, which is given by the difference between the pressure measured at Bi and the pressure measured at Bo, wherein Bi represents a port for introducing a fluid to be treated and Bo represents a port for discharging a fluid passing through a purification column,_achieved by allowing bovine blood to flow at a flow rate of 200 mL/min is from 0.5 to 30 kPa.

**Patentansprüche**

1.  Fasermaterial, das Polymerfasermischungen einer Vielzahl von Typen fester Fasern mit gleicher Querschnittsform umfasst, wobei das Rohmaterial der Polymerfaser aus der aus Polymethylmethacrylat, Polyester, Polyacrylnitril, Polysulfon, Polyethersulfon, Polyarylethersulfon, Polypropylen, Polystyrol, Polycarbonat, Cellulose, Cellulosetriacetat und EthylenVinylalkohol-Copolymer bestehenden Gruppe ausgewählt ist,

    wobei Fasern als Fasern mit gleicher Querschnittsform erachtet werden, wenn der Querschnitt von zwei Fasern verglichen wird und durch den Vergleich festgestellt wird, dass keiner der nachstehenden Punkte (i) bis (vi) erfüllt ist:

    (i) eine der Fasern weist einen Vorsprung am Umfang des Querschnitts auf und die andere Faser weist keinen Vorsprung am Umfang des Querschnitts auf,
    (ii) die Anzahl der Vorsprünge ist unter den Fasern unterschiedlich,
    (iii) wenn die Fasern dieselbe Anzahl an Vorsprüngen aufweisen und eine Differenz der Oberflächenzunahmerate von nicht weniger als 30 %, wie durch das Verfahren gemäß der Beschreibung bestimmt, aufweisen,
    (iv) eine Faser, die einen runden oder ovalen Querschnitt aufweist, unter Fasern, die keinen Vorsprung aufweisen, wird mit einer anderen Faser verglichen, die in ähnlicher Weise einen runden oder ovalen Querschnitt aufweist, und durch den Vergleich wird festgestellt, dass diese Fasern eine Differenz der Oberflächenzunahmerate von nicht weniger als 30 % oder eine Differenz des Modifikationsausmaßes von nicht weniger als 50 %, wie durch das Verfahren gemäß der Beschreibung bestimmt, aufweisen,
    (v) eine Faser, die einen runden oder ovalen Querschnitt aufweist, und eine Faser, die einen eckigen Querschnitt aufweist,
    (vi) wenn jede der Fasern einen eckigen Querschnitt aufweist, weisen die Fasern Querschnitte mit unterschiedlichen Anzahlen an Ecken auf,
    (vii) wenn die Fasern, die eckige Querschnitte mit derselben Anzahl von Ecken aufweisen, eine Differenz der Oberflächenzunahmerate von nicht weniger als 30 % aufweisen,

    wobei der mittlere Porenradius der festen Fasern, wie durch das Verfahren gemäß der Beschreibung bestimmt, im Bereich von 1 bis 100 nm liegt,
    wobei das Zusammensetzungsanteil jedes von zumindest zwei aus der Vielzahl von Typen von Fasern an den Fasermischungen insgesamt nicht weniger als 5,0 % beträgt und, wenn unter dieser Vielzahl von Typen von Fasern mit einem Zusammensetzungsanteil von nicht weniger als 5,0 % Fasern mit der höchsten und niedrigsten Oberflächenzunahmerate, die durch die nachstehende Formel (1) angegeben werden, jeweils als Faser$_{(max)}$ und Faser$_{(min)}$ bezeichnet werden, die Oberflächenzunahmerate von Faser$_{(min)}$ um 3,0 % oder mehr im Vergleich zu jener von Faser$_{(max)}$ verringert ist, wobei der Belegungsanteil Sfo des Umkreises der Faser$_{(min)}$, wie durch die nachstehende Formel (3) angegeben, nicht mehr als 0,90 beträgt, wobei in der Formel (3) Sf für die Querschnittsfläche eines Faserquerschnitts steht und So für die Fläche des Umkreises des Faserquerschnitts steht, wobei das Modifikationsausmaß Do/Di der Faser$_{(min)}$ nicht weniger als 1,10 und nicht mehr als 6,00 beträgt, wobei Do für den Durchmesser des Umkreises des Faserquerschnitts steht und Di für den Durchmesser des Inkreises des Faserquerschnitts steht und die Zusammensetzungsanteile von Faser$_{(max)}$ und Faser$_{(min)}$ an den Fasermischungen insgesamt, wie durch die Formel (2) angegeben, nicht weniger als 30,0 % bzw. nicht weniger als 8,0 % beträgt, und die Faser$_{(max)}$ (a) eine Oberflächenzunahmerate von nicht weniger als 1,20 aufweist und (b) eine poröse Faser mit einer spezifischen Mikroporenoberfläche, die durch die Messung des Ausmaßes der durch Kapillarkondensation induzierten Senkung des Gefrierpunkts von Wasser in Mikroporen mittels Differentialscanningkalorimetrie unter Verwendung eines Differentialscanningkalorimeters erhalten wird, von nicht weniger als 10 m$^2$/g aufweist, wobei das Faserdurchmesserverhältnis Y, das durch Dividieren des äquivalenten Kreisdurchmessers der Faser$_{(min)}$ durch den äquivalenten Kreisdurchmesser der Faser$_{(max)}$ erhalten wird, nicht weniger als 0,2 und nicht mehr als 10,0 beträgt, wobei der äquivalente Kreisdurchmesser der Faser$_{(min)}$ und der äquivalente Kreisdurchmesser der Faser$_{(max)}$ durch die Formel (4) angegeben werden, wobei S in der Formel (4) für die Querschnittsfläche einer Faser steht:

$$\text{Oberflächenzunahmerate} = (\text{Umfang eines Faserquerschnitts})/(\text{Umfang eines Kreises mit derselben Querschnittsfläche wie der Faserquerschnitt}) \qquad (1)$$

$$\text{Zusammensetzungsanteil an Fasermischungsmaterialien insgesamt (\%)} = \text{Anzahl der Fasern mit gleicher Querschnittsform / Gesamtanzahl der Fasern x 100} \qquad (2)$$

$$\text{Belegungsrate Sfo eines Umkreises} = Sf/So \qquad (3)$$

$$\text{Äquivalenter Kreisdurchmesser eines Faserquerschnitts} = 2 \times (S/\pi)^{1/2} \qquad (4).$$

2. Fasermaterial nach Anspruch 1, wobei die Faser$_{(min)}$ eine poröse Faser mit einer spezifischen Mikroporenoberfläche, die durch die Messung des Ausmaßes der durch Kapillarkondensation induzierten Senkung des Gefrierpunkts von Wasser in Mikroporen mittels Differentialscanningkalorimetrie unter Verwendung eines Differentialscanningkalorimeters erhalten wird, von nicht weniger als 5 m$^2$/g ist.

3. Fasermaterial nach Anspruch 2, wobei die Faser$_{(min)}$ eine poröse Faser mit einer spezifischen Mikroporenoberfläche von nicht weniger als 10 m$^2$/g ist.

4. Fasermaterial nach einem der Ansprüche 1 bis 3, wobei die Oberflächenzunahmerate der Faser$_{(min)}$ nicht weniger als 1,10 beträgt.

5. Fasermaterial nach einem der Ansprüche 1 bis 4, wobei die Faserquerschnittsform der Faser$_{(min)}$ rund oder oval ist.

6. Fasermaterial nach einem der Ansprüche 1 bis 5, wobei der äquivalente Kreisdurchmesser, der durch die Formel (4) angegeben wird, der Faser$_{(max)}$ nicht weniger als 10 $\mu$m und nicht mehr als 1.000 $\mu$m beträgt.

7. Fasermaterial nach Anspruch 1 bis 6, wobei das Sfo-Verhältnis Z, das durch Dividieren der Belegungsrate Sfo in dem Umkreis der Faser$_{(max)}$ durch die Belegungsrate Sfo in dem Umkreis der Faser$_{(min)}$ nicht weniger als 0,20 beträgt.

8. Fasermaterial nach einem der Ansprüche 1 bis 7, das aus geraden Fasern besteht.

9. Fasermaterial nach einem der Ansprüche 1 bis 8, wobei die Fasermischung aus zwei Typen von Fasern besteht, die eine gleiche Querschnittsform aufweisen.

10. Fasermaterial nach einem der Ansprüche 1 bis 9, wobei die Faser$_{(max)}$ und die Faser$_{(min)}$ aus demselben Rohmaterial hergestellt sind.

11. Fasermaterial nach Anspruch 9 oder 10, wobei das Rohmaterial ein Estergruppen enthaltendes Polymer ist.

12. Reinigungssäule, die ein Kunststoffgehäuse und ein Fasermaterial nach einem der Ansprüche 1 bis 11 umfasst, wobei das Fasermaterial in dem Kunststoffgehäuse in axialer Richtung gerade angeordnet ist und sich zwischen Öffnungen an beiden Seiten des Gehäuses erstreckt und jeweils an einem Ende des Kunststoffgehäuses eine Einlassöffnung und eine Auslassöffnung für ein zu behandelndes Fluid bereitgestellt sind; und

wobei der äquivalente Durchmesser des Durchflussquerschnitts, der durch die nachstehende Formel (6) angegeben wird, nicht weniger als 10 $\mu$m und nicht mehr als 250 $\mu$m beträgt,

$$D_p = 4 \times ((D_{Gehäuse}/2)^2 - (D_{Faser-max}/2)^2 \times N_{max} - (D_{Faser-min}/2)^2 \times N_{min}) / (D_{Gehäuse} + D_{Faser-max}$$

$$\times SA_{max} \times N_{max} + D_{Faser-min} \times SA_{min} \times N_{min}) \qquad (6),$$

wobei in der Formel (6) $D_{Gehäuse}$ für den Innendurchmesser des Säulengehäuses steht und $D_{Faser-max}$ für den äquivalenten Kreisdurchmesser einer Faser$_{(max)}$ steht, $D_{Faser-min}$ für den äquivalenten Kreisdurchmesser einer Faser$_{(min)}$ steht, $N_{max}$ für die Anzahl an Fasern$_{(max)}$ steht, $N_{min}$ für die Anzahl an Fasern$_{(min)}$ steht, $SA_{max}$ für die Oberflächenzunahmerate einer Faser$_{(max)}$ steht und $SA_{min}$ für die Oberflächenzunahmerate einer Faser$_{(min)}$ steht; und

wobei der Druckverlust, der durch die Differenz zwischen dem an Bi gemessenen Druck und dem an Bo gemessenen Druck angegeben wird, wobei Bi für eine Öffnung zum Einbringen eines zu behandelnden Fluids steht und Bo für eine Öffnung zur Abgabe eines die Reinigungssäule passierenden Fluids steht, und der erzielt wird, indem Rinderblut mit einer Durchflussrate von 200 ml/min fließen gelassen wird, 0,5 bis 30 kPa beträgt.

**Revendications**

1. Matériau fibreux comprenant des mélanges de fibres polymères présentant une pluralité de types de fibres solides présentant une forme de section transversale commune, dans lequel le matériau brut de la fibre polymère est sélectionné dans le groupe consistant en polyméthylméthacrylate, polyester, polyacrylonitrile, polysulfone, polyéthersulfone, polyaryléthersulfone, polypropylène, polystyrène, polycarbonate, de cellulose, triacétate de cellulose et copolymère d'éthylène-alcool de vinyle,

dans lequel des fibres sont considérées comme présentant une forme de section transversale commune lorsque les sections transversales de deux fibres sont comparées et que la comparaison identifie qu'aucun des points (i) à (vi) suivants ne se vérifie,

(i) l'une des fibres présente une saillie à la périphérie de la section transversale et l'autre fibre ne présente pas de saillie à la périphérie de la section transversale,
(ii) le nombre de saillies est différent entre les fibres,
(iii) lorsque les fibres présentent le même nombre de saillies et, présentent une différence d'au moins 30 % dans le taux d'augmentation de surface comme déterminé par le procédé de la description,
(iv) une fibre présentant une section transversale ronde ou ovale, parmi des fibres ne présentant pas de saillie, est comparée à une autre fibre présentant de manière similaire une section transversale ronde ou ovale et la comparaison identifie ces fibres comme présentant une différence d'au moins 30 % dans le taux d'augmentation de surface ou comme présentant une différence d'au moins 50 % dans le degré de modification comme déterminé par le procédé de la description,
(v) une fibre présentant une section transversale ronde ou ovale et une fibre présentant une section transversale angulaire,
(vi) lorsque chacune des fibres présente une section transversale angulaire, les fibres présentent des sections transversales avec différents nombres d'angles,
(vii) lorsque les fibres présentent des sections transversales angulaires avec le même nombre d'angles et, présentent une différence d'au moins 30 % dans le taux d'augmentation de surface, dans lequel le rayon de pore moyen des fibres solides est dans la plage de 1 à 100 nm comme déterminé par le procédé de la description,

dans lequel le rapport de composition de chacun d'au moins deux parmi la pluralité de types de fibres sur le total des mélanges de fibres est d'au moins 5,0 %, et lorsque, parmi cette pluralité de types de fibres présentant un rapport de composition d'au moins 5,0 %, les fibres présentant les taux d'augmentation de surface les plus élevés et les plus bas, qui sont donnés par la formule (1) ci-dessous, sont respectivement désignées par la fibre$_{(max)}$ et la fibre$_{(min)}$, le taux d'augmentation de surface de la fibre$_{(min)}$ est réduit de 3,0 % ou plus comparé à celui de la fibre$_{(max)}$, dans lequel le taux d'occupation $Sfo$ par rapport au cercle circonscrit de la fibre$_{(min)}$, qui est donné par la formule (3) ci-dessous, est d'au plus 0,90, dans lequel dans la formule (3) $Sf$ représente la surface de section transversale d'une section transversale de fibre et $So$ représente la zone du cercle circonscrit de la section transversale de fibre, dans lequel le degré de modification $Do/Di$ de la fibre$_{(min)}$ est d'au moins 1,10 et d'au plus 6,00, dans lequel *Do* représente le diamètre du cercle circonscrit d'une section transversale de fibre et *Di* représente le diamètre du cercle inscrit de la section transversale de fibre, et les rapports de composition de la fibre$_{(max)}$ et de la fibre$_{(min)}$ sur le total des mélanges de fibres, qui sont donnés par la formule (2), sont d'au moins 30,0 % et d'au moins 8,0 %, respectivement, et la fibre$_{(max)}$ présente (a) un taux d'augmentation de surface d'au moins 1,20 et est (b) une fibre poreuse avec une surface spécifique de micropores, qui est obtenue par le biais de la mesure du degré d'abaissement du point de congélation de l'eau dans les micropores induit par condensation capillaire par calorimétrie différentielle à balayage à l'aide d'un calorimètre différentiel à balayage, d'au moins 10 m$^2$/g, dans lequel le rapport de diamètre des fibres Y, qui est obtenu en divisant le diamètre de cercle équivalent de la fibre$_{(min)}$ par le diamètre de cercle équivalent de la fibre$_{(max)}$, est d'au moins 0,2 et d'au plus 10,0, dans lequel le diamètre de cercle équivalent de la fibre$_{(min)}$ et le diamètre de cercle équivalent de la fibre$_{(max)}$ sont donnés par la formule (4), dans lequel S dans la formule (4) représente la surface de section transversale d'une fibre ; dans lequel

```
taux d'augmentation de surface = (la circonférence
d'une section transversale de fibre)/(la circonférence d'un
cercle présentant la même surface de section transversale
que la section transversale de fibre)   (1)

rapport de composition dans le total de matériaux de
mélange de fibres (%) = le nombre de fibres présentant une
forme de section transversale commune/ nombre total de
fibres x 100   (2)

taux d'occupation Sfo par rapport à un cercle
circonscrit = Sf/So (3)

diamètre de cercle équivalent d'une section
transversale de fibre = 2 x (S/π)^{1/2}   (4).
```

2. Matériau fibreux selon la revendication 1, dans lequel la fibre$_{(min)}$ est une fibre poreuse avec une surface spécifique de micropores, qui est obtenue par le biais de la mesure du degré d'abaissement du point de congélation de l'eau dans les micropores induit par condensation capillaire par calorimétrie différentielle à balayage à l'aide d'un calorimètre différentiel à balayage, d'au moins 5 m$^2$ / g.

3. Matériau fibreux selon la revendication 2, dans lequel la fibre$_{(min)}$ est une fibre poreuse avec une surface spécifique de micropores d'au moins 10 m$^2$/g.

4. Matériau fibreux selon l'une quelconque des revendications 1 à 3, dans lequel le taux d'augmentation de surface de la fibre$_{(min)}$ est d'au moins 1,10.

5. Matériau fibreux selon l'une quelconque des revendications 1 à 4, dans lequel la forme de section transversale de fibre de la fibre$_{(min)}$ est ronde ou ovale.

6. Matériau fibreux selon l'une quelconque des revendications 1 à 5, dans lequel le diamètre de cercle équivalent, qui est donné par la formule (4), de la fibre$_{(max)}$ est d'au moins 10 $\mu$m et d'au plus 1000 $\mu$m.

7. Matériau fibreux selon l'une quelconque des revendications 1 à 6, dans lequel le rapport des *Sfo* Z obtenu en divisant le taux d'occupation *Sfo* par rapport au cercle circonscrit de la fibre$_{(max)}$ par le taux d'occupation *Sfo* par rapport au cercle circonscrit de la fibre$_{(min)}$ est d'au moins 0,20.

8. Matériau fibreux selon l'une quelconque des revendications 1 à 7, qui est composé de fibres rectilignes.

9. Matériau fibreux selon l'une quelconque des revendications 1 à 8, dans lequel le mélange de fibres est composé de deux types de fibres présentant une forme de section transversale commune.

10. Matériau fibreux selon l'une quelconque des revendications 1 à 9, dans lequel la fibre$_{(max)}$ et la fibre$_{(min)}$ sont produites à partir du même matériau brut.

11. Matériau fibreux selon la revendication 9 ou 10, dans lequel le matériau brut est un polymère contenant un groupe ester.

12. Colonne de purification comprenant un carter en plastique et le matériau fibreux selon l'une quelconque des revendications 1 à 11, dans lequel le matériau fibreux est agencé dans le carter en plastique de manière rectiligne dans la direction axiale s'étendant entre les ouvertures des deux côtés du carter, et un orifice d'entrée et un orifice de sortie pour un fluide qui doit être traité sont prévus à chaque extrémité du carter en plastique ; et

dans lequel le diamètre équivalent de section transversale d'écoulement, qui est donné par la formule (6) ci-dessous, est d'au moins 10 μm et d'au plus 250 μm,

$$D_p = 4 \times ((D_{carter}/2)^2 - (D_{fibre-max}/2)^2 \times N_{max} - (D_{fibre-min}/2)^2 \times N_{min})/(D_{carter} + D_{fibre-max} \times SA_{max} \times N_{max} + D_{fibre-min} \times SA_{min} \times N_{min}) \quad (6),$$

dans lequel dans la formule (6), $D_{boîtier}$ représente le diamètre interne d'un carter de colonne, et $D_{fibre-max}$ représente le diamètre de cercle équivalent d'une fibre$_{(max)}$, $D_{fibre-min}$ représente le diamètre de cercle équivalent d'une fibre$_{(min)}$, $N_{max}$ représente le nombre de fibres $_{(max)}$, $N_{min}$ représente le nombre de fibres$_{(min)}$, $SA_{max}$ représente le taux d'augmentation de surface d'une fibre$_{(max)}$ et $SA_{min}$ représente le taux d'augmentation de surface d'une fibre$_{(min)}$ ; et
dans lequel la perte de pression, qui est donnée par la différence entre la pression mesurée au niveau de Bi et la pression mesurée au niveau de Bo, dans lequel Bi représente un orifice d'introduction d'un fluide devant être traité et Bo représente un orifice d'évacuation d'un fluide passant à travers une colonne de purification, obtenue en laissant du sang bovin s'écouler à un débit de 200 mL/min est de 0,5 à 30 kPa.

[Fig. 1]

[Fig. 2]

[Fig. 3]

[Fig. 4]

[Fig. 5]

[Fig. 6]

[Fig. 7]

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2011156022 A
- JP 2010148851 A
- JP 2002220758 A
- JP 2004263341 A
- JP 2002194621 A
- JP 2012115743 A
- JP 2008155009 A
- JP 2000225304 A
- US 2009275874 A
- US 2015071659 A

**Non-patent literature cited in the description**

- **KAZUHIKO ISHIKIRIYAMA et al.** *JOURNAL OF COLLOID AND INTERFACE SCIENCE,* 1995, vol. 171, 103-111